(19) Europäisches Patentamt
European Patent Office
Office européen des brevets

(11) EP 1 762 251 A1

(12) # EUROPEAN PATENT APPLICATION

(43) Date of publication:
**14.03.2007 Bulletin 2007/11**

(51) Int Cl.:
***A61L 2/08*** *(2006.01)* ***A61L 2/00*** *(2006.01)*

(21) Application number: **06020540.8**

(22) Date of filing: **23.03.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **23.03.2000 US 533547**

(62) Document number(s) of the earlier application(s) in accordance with Art. 76 EPC:
**01932512.5 / 1 299 131**

(71) Applicant: **Clearant, Inc.**
**Los Angeles, CA 90025 (US)**

(72) Inventors:
- **Kent, Randall, S.**
**Los Angeles**
**California 90025 (US)**
- **Horton, Edward, A.**
**Los Angeles**
**California 90025 (US)**
- **MacPhee, Martin, J.**
**Los Angeles**
**California 90025 (US)**
- **Beall, Dawson**
**Los Angeles**
**California 90025 (US)**

(74) Representative: **Cripps, Joanna Elizabeth et al**
**Mewburn Ellis LLP**
**York House**
**23 Kingsway**
**London WC2B 6HP (GB)**

Remarks:
This application was filed on 29.09.2006 as a divisional application to the application mentioned under INID code 62.

(54) **Methods for sterilizing biological materials**

(57) Methods are disclosed for sterilizing biological products to reduce the level of active biological contaminants such as viruses, bacteria, yeasts, molds, mycoplasmas and parasites.



Printed by Jouve, 75001 PARIS (FR)

## Description

### *Field of the Invention*

The present invention relates to methods for sterilizing biological materials to reduce the level of active biological contaminants therein, such as viruses, bacteria, yeasts, molds, mycoplasmas and/or parasites.

### *Background of the Invention*

Several products that are prepared from human, veterinary or experimental use may contain unwanted and potentially dangerous contaminants such as viruses, bacteria, yeasts, molds, mycoplasmas and parasites. Consequently, it is of utmost importance that any biologically active contaminant in the product be inactivated before the product is used. This is especially critical when the product is to be administered directly to a patient, for example in blood transfusions, organ transplants and other forms of human therapies. This is also critical for various biotechnology products which are grown in media which contain various types of plasma and which may be subject to mycoplasma or other viral contaminants.

Previously, most procedures have involved methods that screen or test products for a particular contaminant rather than removal or inactivation of the contaminant from the product. Products that test positive for a contaminant are merely not used. Examples of screening procedures include the testing for a particular virus in human blood from blood donors. Such procedures, however, are not always reliable and are not able to detect the presence of viruses in very low numbers. This reduces the value or certainty of the test in view of the consequences associated with a false negative result. False negative results can be life threatening in certain cases, for example in the case of Acquired Immune Deficiency Syndrome (AIDS). Furthermore, in some instances it can take weeks, if not months, to determine whether or not the product is contaminated.

More recent efforts have focused in methods to remove or inactivate contaminants in the products. Such methods include heat treating, filtration and the addition of chemical inactivants or sensitizers to the product. Heat treatment requires that the product be heated to approximately 60°C for about 70 hours which can be damaging to sensitive products. Heat inactivation can destroy up to 50% of the biological activity of the product. Filtration involves filtering the product in order to physically remove contaminants. Unfortunately this method may also remove products that have a high molecular weight. Further, in certain cases small viruses may not be removed by the filter because of the larger molecular structure of the product. The procedure of chemical sensitization involves the addition of noxious agents which bind to the DNA/RNA of the virus and which are activated either by UV or ionizing radiation to produce free radicals which break the chemical bonds in the backbone of the DNA/RNA of the virus or complex it in such a way that the virus can no longer replicate. This procedure requires that unbound sensitizer is washed from cellular products since the sensitizers are toxic, if not mutagenic or carcinogenic, and can not be administered to a patient.

Irradiating a product with gamma radiation is another method of sterilizing a product. Gamma radiation is effective in destroying viruses and bacteria when given in high total doses (Keathly et al., "Is There Life After Irradiation? Part 2," BioPharm July-August, 1993, and Leitman, USe of Blood Cell Irradiation in the Prevention of Post Transfusion Graft-vs-Host Disease," Transfusion Science 10:219-239 (1989)). The published literature in this area, however, teaches that gamma radiation can be damaging to radiation sensitive products, such as blood. In particular, it has been shown that high radiation doses are injurious to red cells, platelets and granulocytes (Leitman). U.S. Patent No. 4,620,908 discloses that protein products must be frozen prior to irradiation in order to maintain the viability of the protein product. This patent concludes that "[i]f the gamma irradiation were applied while the protein material was at, for example, ambient temperature, the material would be also completely destroyed, that is the activity of the material would be rendered so low as to be virtually ineffective." Unfortunately, many sensitive biologicals, such as blood, would lose viability and activity if subjected to freezing for irradiation purposes and then thawing prior to administration to a patient.

In view of the difficulties discussed above, there remains a need for methods of sterilizing biological materials that are effective for reducing the level of active biological contaminants without an adverse effect on the biological material.

### *Summary of the Invention*

Accordingly, it is an object of the present invention to provide methods of sterilizing biological materials by reducing the level of active biological contaminants without adversely effecting the biological material. Other objects, features and advantages of the present invention will be set forth in the detailed description of preferred embodiments that follows, and in part will be apparent from the description or may be learned by practice of the invention. These objects and advantages of the invention will be realized and attained by the compositions and methods particularly pointed out in the written description and claims hereof.

In accordance with these and other objects, a first embodiment of the present invention is directed to a method for sterilizing a biological material that is sensitive to ionizing radiation comprising: (i) reducing the residual solvent content of a biological material to a level effective to protect the biological material from ionizing radiation; and (ii) irradiating the biological material with radiation at an effective rate for a time effective to sterilize the biological material.

A second embodiment of the present invention is directed to a method for sterilizing a biological material that is sensitive to ionizing radiation comprising: (i) adding to a biological material at least one stabilizer in an amount effective to protect the biological material from ionizing radiation; and (ii) irradiating the biological material with radiation at an effective rate for a time effective to sterilize the biological material.

A third embodiment of the present invention is directed to a method for sterilizing a biological material that is sensitive to ionizing radiation comprising:

(i) reducing the residual solvent content of a biological material to a level effective to protect the biological material from ionizing radiation; (ii) adding to the biological material at least one stabilizer in an amount effective to protect the biological material from ionizing radiation; and (iii) irradiating the biological material with radiation at an effective rate for a time effective to sterilize the biological material. According to this embodiment, steps (i) and (ii) may be reversed.

***Description of the Figures***

FIGURES 1 and 2 are graphs showing the protective effects of certain stabilizers on lyophilized anti-insulin monoclonal antibody exposed to 45 kGy of low dose gamma irradiation.

FIGURES 3A-3C are graphs showing the protective effects of certain stabilizers on lyophilized anti-insulin monoclonal antibody exposed to 45 kGy of low dose gamma irradiation.

FIGURE 4 is a graph showing the protective effects of primary lyophilizing and secondary lyophilizing on the sensitivity of a monoclonal antibody.

FIGURE 5 is a graph showing the protective effect of freeze-drying and/or an added stabilizer on the activity of Factor VIII.

FIGURE 6 is a graph showing the protective effects of certain stabilizers on liquid or lyophilized antithrombin III exposed to 25 kGy of low dose gamma irradiation.

FIGURES 7-14 are graphs showing the protective effect of certain stabilizers on the activity of lyophilized anti-insulin monoclonal antibody.

FIGURE 15 is a graph showing the protective effect of stabilizers on the activity of lyophilized anti-insulin monoclonal antibody when the sample was irradiated at a high dose rate (30 kGy/hr).

FIGURE 16 is a graph showing the effect of a stabilizer on lyophilized thrombin that was irradiated with gamma radiation.

FIGURE 17 is a graph showing the effect of a stabilizer on IgM activity after irradiation with gamma radiation.

FIGURE 18 is a chromatogram showing the effects of gamma irradiation on albumin.

FIGURE 19 is a graph showing the protective effects of lyophilization and/or the presence of a stabilizer on thrombin activity after irradiation with gamma radiation.

FIGURES 20-25 are graphs showing the protective effects of certain stabilizers on liquid IVIG polyclonal antibody exposed to 45 kGy of gamma irradiation (1.8 kGy/hr).

FIGURE 26 is a graph showing the effects of pH on the recovery of urokinase (liquid or lyophilized) irradiated in the presence of a stabilizer

***Detailed Description of the Preferred Embodiments***

***A. Definitions***

Unless defined otherwise, all technical and scientific terms used herein are intended to have the same meaning as is commonly understood by one of ordinary skill in the relevant art. All patents and publications mentioned herein are expressly incorporated by reference.

As used herein, the term "biological material" is intended to mean any substance derived or obtained from a living organism. Illustrative examples of biological materials include, but are not limited to, the following: cells; tissues; blood or blood components; proteins, including recombinant and transgenic proteins; botanicals; foods and the like. Preferred examples of biological materials include, but are not limited to, the following: ligaments; tendons; nerves; bone, including demineralized bone matrix, grafts, joints, femurs, femoral heads, etc.; teeth; skin grafts; bone marrow, including bone marrow cell suspensions, whole or processed; heart valves; cartilage; corneas; arteries and veins; organs for transplant, such as hearts, lungs, liver, kidney, intestine, pancreas, limbs and digits; lipids; carbohydrates; collagen (native, afibrillar, atelomeric, soluble and insoluble); chitin and its derivatives including chitosan and its derivatives including NO-carboxy chitosan (NOCC); stem cells, islet of langerhans cells, and other cellular transplants, including genetically altered cells; red blood cells; white blood cells, including monocytes and stem cells; and platelets.

As used herein, the term "sterilize" is intended to mean a reduction in the level of at least one active biological contaminant found in the biological material being treated according to the present invention.

As used herein, the term "biological contaminant" is intended to mean a contaminant that, upon direct or indirect contact with a biological material, may have a deleterious effect on a biological material. Such biological contaminants include the various viruses, bacteria and parasites known to those of skill in the art to generally be found in or infect biological materials such as whole blood or blood components. Examples of biological contaminants include, but are not limited to, the following: viruses, such as human immunodeficiency viruses and other retroviruses, herpes viruses, paramyxoviruses, cytomegaloviruses, hepatitis viruses (including hepatitis B and hepatitis C), pox viruses, toga viruses, Ebstein-Barr virus and parvoviruses; bacteria, such as *Escherichia, Bacillus, Campylobacter, Streptococcus* and *Staphalococcus;* parasites, such as *Trypanosoma* and malarial parasites, including *Plasmodium* species; yeasts; molds; mycoplasmas; and prions. As used herein, the term "active biological contaminant" is intended to mean a biological contaminant that is capable of causing the deleterious effect.

As used herein, the term "blood components" is intended to mean one or more of the components that may be separated from whole blood and include, but are not limited to, cellular blood components, such as red blood cells, white blood cells and platelets; blood proteins, such as blood clotting factors, enzymes, albumin, plasminogen, fibrinogen and immunoglobulins; and liquid blood components, such as plasma and plasma-containing compositions.

As used herein, the term "cellular blood component" is intended to mean one or more of the components of whole blood that comprises cells, such as red blood cells, white blood cells or platelets.

As used herein, the term "blood protein" is intended to mean one or more of the proteins that are normally found in whole blood. Illustrative examples of blood proteins found in mammals (including humans) include, but are not limited to, coagulation proteins (both vitamin K-dependent, such as Factor VII or Factor IX, and non-vitamin K-dependent, such as Factor VIII and von Willebrands factor), albumin, lipoproteins (high density lipoproteins and/or low density lipoproteins), complement proteins, globulins (such as immunoglobulins IgA, IgM, IgG and IgE), and the like. A preferred group of blood proteins include Factor I (Fibrinogen), Factor II (Prothrombin), Factor III (Tissue Factor), Factor IV (Calcium), Factor V (Proaccelerin), Factor VI (Accelerin), Factor VII (Proconvertin, serum prothrombin conversion), Factor VIII (Antihemophiliac factor A), Factor IX (Antihemophiliac factor B), Factor X (Stuart-Prower Factor), Factor XI (Plasma thromboplastin antecedent), Factor XII (Hageman Factor), Factor XIII (Protansglutamidase), von Willebrand Factor (vWF), Factor Ia, Factor IIa, Factor Va, Factor VIa, Factor VIIa, Factor VIIIa, Factor IXa, Factor Xa, and Factor XIIIa.

As used herein, the term "liquid blood component" is intended to mean one or more of the fluid, non-cellular components of whole blood, such as plasma (the fluid, non-cellular portion of the blood of humans or animals as found prior to coagulation) or serum (the fluid, non-cellular portion of the blood of humans or animals after coagulation).

As used herein, the term "a biologically compatible solution" is intended to mean a solution to which biological materials may be exposed, such as by being suspended or dissolved therein, and remain viable, i.e., retain their essential biological and physiological characteristics. Such biologically compatible solutions preferably contain an effective amount of at least one anticoagulant.

As used herein, the term "a biologically compatible buffered solution" is intended to mean a biologically compatible solution having a pH and osmotic properties (e.g, tonicity, osmolality and/or oncotic pressure) suitable for maintaining the integrity ofbiological materials. Suitable biologically compatible buffered solutions typically have a pH between 5 and 8.5 and are isotonic or only moderately hypotonic or hypertonic. Biologically compatible buffered solutions are known and readily available to those of skill in the art.

As used herein, the term "stabilizer" is intended to mean a compound or material that reduces any damage to the biological material being irradiated to a level that is insufficient to preclude the safe and effective use of that material. Illustrative examples of stabilizers include, but are not limited to, the following: antioxidants, such as ascorbic acid and tocopherol; and free radical scavengers, such as ethanol. Preferred examples of stabilizers include, but are not limited to, the following: fatty acids, including 6,8-dimercapto-octanoic acid (lipoic acid) and its derivatives and analogues (alpha, beta, dihydro, bisno and tetranor lipoic acid), thioctic acid, 6,8-dimercapto-octanoic acid, dihydrolopoate (DL-6,8-dithioloctanoic acid methyl ester), lipoamide, bisonor methyl ester and tatranordihydrolipoic acid, furan fatty acids, oleic and linoleic and palmitic acids and their salts and derivatives; flavonoids, phenylpropaniods, and flavenols, such as quercetin, rutin and its derivatives, apigenin, aminoflavone, catechin, hesperidin and, naringin; carotenes, including beta-carotene; Co-Q10; xanthophylls; polyhydric alcohols, such as glycerol, mannitol; sugars, such as xylose, glucose, ribose, mannose, fructose and trehalose; amino acids, such as histidine, N-acetylcysteine (NAC), glutamic acid, tryptophan, sodium carpryl N-acetyl tryptophan and methionine; azides, such as sodium azide; enzymes, such as Superoxide Dismutase (SOD) and Catalase; uric acid and its derivatives, such as 1,3-dimethyluric acid and dimethylthiourea; allopurinol; thiols, such as glutathione and cysteine; trace elements, such as selenium; vitamins, such as vitamin A, vitamin C (including its derivatives and salts such as sodium ascorbate and palmitoyl ascorbic acid) and vitamin E (and its derivatives and salts such as tocopherol acetate and alpha-tocotrienol); chromanol-alpha-C6; 6-hydroxy-2,5,7,8-tetramethylchroma-2 carboxylic acid (Trolox) and derivatives; extraneous proteins, such as gelatin and albumin; tris-3-methyl-1-phenyl-2-pyrazolin-5-one (MCI-186); citiolone; puercetin; chrysin; dimethyl sulfoxide (DMSO); piperazine diethanesulfonic acid (PIPES); imidazole; methoxypsoralen (MOPS); 1,2-dithiane-4,5-diol; reducing substances, such as butylated hydroxyanisole (BHA) and butylated hydroxytoluene (BHT); cholesterol; probucol; indole derivatives; thimerosal; lazaroid and tirilazad mesylate; proanthenols; proanthocyanidins; ammonium sulfate; Pegorgotein (PEG-SOD); N-*tert*-butyl-alpha-phenylnitrone (PBN); and 4-nydroxy-2,2,6,6-Tetramethylpiperidin-1-oxyl (Tempol)

As used herein, the term "residual solvent content" is intended to mean the amount of freely-available liquid in the biological material. Freely-available liquid means that liquid, such as water or an organic solvent (*e.g.* ethanol, isopropanol, polyethylene glycol, etc.), present in the biological material that is not bound to or complexed with one or more of the non-liquid components of the biological material (e.g. proteins, metal ions or salts, etc.). Freely-available liquid includes intracellular water. The residual solvent contents referenced herein refer to levels determined by the FDA approved, modified Karl Fischer method (Meyer and Boyd, Analytical Chem., 31, 215-219, 1959; May, et al., J. Biol. Standardization, 10, 249-259, 1982; Centers for Biologics Evaluation and Research, FDA, Docket No. 89D-0140, 83-93; 1990).

As used herein, the term "sensitizer" is intended to mean a substance that selectively targets viral, bacterial, and/or parasitic contaminants, rendering them more sensitive to inactivation by radiation, therefore permitting the use of a lower rate of radiation and/or a shorter time of irradiation than in the absence of the sensitizer. Illustrative examples of suitable sensitizers include, but are not limited to, the following: psoralen and its derivatives and analogs (including 3-carboethoxy psoralens); angelicins, khellins and coumarins which contain a halogen substituent and a water solubilization moiety, such as quaternary ammonium ion or phosphonium ion; nucleic acid binding compounds; brominated hematoporphyrin; phthalocyanines; purpurins; porphorins; halogenated or metal atom-substituted derivatives of dihematoporphyrin esters, hematoporphyrin derivatives, benzoporphyrin derivatives, hydrodibenzoporphyrin dimaleimade, hydrodibenzoporphyrin, dicyano disulfone, tetracarbethoxy hydrodibenzoporphyrin, and tetracarbethoxy hydrodibenzoporphyrin dipropionamide; doxorubicin and daunomycin, which may be modified with halogens or metal atoms; netropsin; BD peptide, S2 peptide; S-303 (ALE compound); dyes, such as hypericin, methylene blue, eosin, fluoresceins (and their derivatives), flavins, merocyanine 540; photoactive compounds, such as bergapten; and SE peptide.

As used herein, the term "proteinaceous material" is intended to mean a cellular material that comprises at least one protein or peptide. This material is preferably composed primarily of protein(s) and/or peptide(s). It may be a naturally occurring material, either in its native state or following processing/purification and/or derivatization. It may be artificially produced, either by chemical synthesis or utilizing recombinant/transgenic technology. Such artificially produced material may also be processed/purified and/or derivatized. Illustrative examples of proteinaceous materials include, but are not limited to, the following: proteins/peptides produced from tissue culture; milk (dairy products); ascites; hormones; growth factors; materials, including pharmaceuticals, extracted or isolated from animal tissue (such as heparin and insulin) or plant matter; plasma (including fresh, frozen and freeze-dried); fibrinogen, fibrin and/or fibrin sealant products; whole blood; protein C; protein S; alpha-1 anti-trypsin (alpha-1 protease inhibitor); butyl-cholinesterase; anticoagulants, such as coumarin drugs (warfarin); streptokinase; tissue plasminogen activator (TPA); erythropoietin (EPO); urokinase; neupogen; antithrombin-3; alpha-glucosidase; (Fetal) Bovine Serum/Horse Serum; meat; immunoglobulins, including anti-sera, monoclonal antibodies, polyclonal antibodies and genetically engineered or produced antibodies; albumin; alpha-globulins; beta-globulins; gamma-globulins; coagulation proteins; complement proteins; and interferons.

As used herein, the term "ionizing radiation" is intended to mean radiation of sufficient energy to ionize (produce ions) the irradiated biological material. Types of ionizing radiation include, but are not limited to, the following: (i) corpuscular (streams of subatomic particles such as neutrons, electrons, and/or protons); and (ii) electromagnetic (originating in a varying electromagnetic field, such as radio waves, visible and invisible light, x-radiation, and gamma rays).

### *B. Particularly Preferred Embodiments*

A first preferred embodiment of the present invention is directed to a method for sterilizing a biological material that is sensitive to ionizing radiation comprising: (i) reducing the residual solvent content of a biological material to a level effective to protect the biological material from ionizing radiation; and (ii) irradiating the biological material with radiation at an effective rate for a time effective to sterilize the biological material.

A second embodiment of the present invention is directed to a method for sterilizing a biological material that is sensitive to ionizing radiation comprising:

(i) adding to a biological material at least one stabilizer in an amount effective to protect the biological material from ionizing radiation; and (ii) irradiating the biological material with radiation at an effective rate for a time effective to sterilize the biological material.

A third embodiment of the present invention is directed to a method for sterilizing a biological material that is sensitive to ionizing radiation comprising:

(i) reducing the residual solvent content of a biological material to a level effective to protect the biological material from ionizing radiation; (ii) adding to the biological material at least one stabilizer in an amount effective to protect the biological material from ionizing radiation; and (iii) irradiating the biological material with radiation at an effective rate for a time effective to sterilize the biological material. The order of steps (i) and (ii) may, of course, be reversed as desired.

The biological material sterilized in accordance with the methods of the present invention may be any material obtained or derived from a living or deceased organism, including a solid material or liquid material or a suspension of any solid(s) in any liquid(s) or a coating of any solid or liquid on a biological or non-biological substrate.

According to the methods of the present invention, the residual solvent content of the biological material is reduced prior to irradiation of the biological material with ionizing radiation. The residual solvent content is reduced to a level that is effective to protect the biological material from the ionizing radiation. Suitable levels of residual solvent content may vary depending upon the nature and characteristics of the particular biological material being irradiated and can be determined empirically by one skilled in the art. Preferably, when the solvent is water, the residual solvent content is less than about 2.0%, more preferably less than about 1.0%, even more preferably less than about 0.5% and most preferably less than about 0.2%.

While not wishing to be bound by any theory of operability, it is believed that the reduction in residual solvent content reduce the degrees of freedom of the biological material and thereby protects it from the effects of the ionizing radiation. Similar results might therefore be achieved by lowering the temperature of the biological material below its eutectic point or below its freezing point to likewise reduce the degrees of freedom of the biological material. These results permit the use of a higher rate of irradiation than might otherwise be acceptable.

The residual solvent content of the biological material may be reduced by any of the methods and techniques known to those skilled in the art for removing solvent from a biological material. A particularly preferred method for reducing the residual solvent content of a biological material is lyophilization. According to a particularly preferred embodiment of the present invention, a biological material which has been lyophilized is stored under vacuum or an inert atmosphere (preferably a noble gas, such as helium or argon, more preferably a higher molecular weight noble gas, and most preferably argon) prior to irradation.

The ionizing radiation employed in the present invention may be any ionizing radiation effective for the inactivation of one or more biological contaminants of the biological material being treated. Preferably the ionizing radiation is electromagnetic radiation and a particularly preferred form ofionizing radiation is gamma radiation.

According to the methods of the present invention, the biological material is irradiated with the ionizing radiation at a rate effective for the inactivation of one or more biological contaminants of the biological material. Suitable rates of irradiation may vary depending upon the particular form of ionizing radiation and the nature and characteristics of the particular biological material being irradiated and the particular biological contaminants being inactivated. Suitable rates of irradiation can be determined empirically by one skilled in the art. Preferably, the rate of irradiation is constant for the duration of the sterilization procedure.

According to a particularly preferred embodiment of the present invention, the rate of irradiation is not more than about 3.0 kGy/hour, more preferably between about 0.1 kGy/hr. and 3.0 kGy/hr, even more preferably between about 0.25 kGy/hr and 2.0 kGy/hour, still even more preferably between about 0.5 kGy/hr and 1.5 kGy/hr and most preferably between about 0.5 kGy/hr and 1.0 kGy/hr.

According to another particularly preferred embodiment of the present invention, the rate of irradiation is at least about 3.0 kGy/hr., more preferably at least about 6 kGy/hr., even more preferably at least about 16 kGy/hr., and most preferably at least about 30 kGy/hr.

The biological material is irradiated with the ionizing radiation for a time effective for the inactivation of one or more biological contaminants of the biological material. Suitable ionization times may vary depending upon the particular form and rate of ionizing radiation and the nature and characteristics of the particular biological material being irradiated and the particular biological contaminants being inactivated. Suitable ionization times can be determined empirically by one skilled in the art.

Optionally, an effective amount of at least one sensitizer is added to the biological material prior to irradiation with ionizing radiation. Suitable sensitizers are known to those skilled in the art.

According to methods of the present invention, the irradiation of the biological material may occur at any temperature which is not deleterious to the biological material being treated. According to a preferred embodiment, the biological material is irradiated at ambient temperature. According to an alternate preferred embodiment, the biological material is irradiated at reduced temperature, preferably at or below the eutectic point of the biological material.

### *C. Examples*

The following examples are illustrative, but not limiting, of the present invention. Other suitable modifications and adaptations are of the variety normally encountered by those skilled in the art and are fully within the spirit and scope of the present invention.

#### *Example 1- Sterilization of Blood*

A 200 ml bag of one day old packed red blood cells was used. Ethanol was added to the cells in order to achieve a final ethanol concentration of 0.01% v/v. The red blood cells were diluted by a factor of one in ten using a modified Citrate Phosphate Dextrose (CPD) solution having a pH of about 6.4 to 6.7 and having the following composition in a total volume of 500 ml:

| | |
|---|---|
| Citrate Acid Monohydrate | 0.2g |
| Sodium Citrate Dihydrate | 27.3g |
| Sodium Monobasic Phosphate | 2.2g |
| Sodium Dibasic Phosphate | 1.0g |
| Dextrose | 3.2g |

The cells were irradiated in a commercial size gamma irradiator which contained a cobalt 60 source rack. Irradiation was done off carrier in an unprotected box. The cells were irradiated for twenty-four hours at a rate of approximately 1 kGy/hr. After the irradiation period the red blood cells were examined visually and were found to be viable, having a brilliant red color. A control sample, consisting of packed red blood cells that were not diluted with the above-described CPD solution, was not viable after irradiation.

Four days after the irradiation procedure, the diluted cells were tested for levels of various blood components and the results are shown in Table 1. The control sample consisted of blood from the same bag as the test sample but it did not undergo irradiation. Table 1 illustrates that dilution and irradiation of human blood cells did not significantly alter the white blood cell count. The platelet count and hematocrit values were slightly lower than the control; however, these values are still within the range that is seen in normal adult blood. The level of hemoglobin was higher than in the control indicating that some red blood cells did lyse during the procedure. This is also evidenced by the lower red blood cell count. Nevertheless, contrary to what has been previously published, up to 50 kGy of radiation did not destroy the components of blood by the present procedure. The cells were also counted and found to be viable after 25 kGy of gamma irradiation delivered at a low dose rate of 1 kGy/hr.

TABLE 1

| Component | Irradiated Blood | Control Blood |
|---|---|---|
| White Blood Cells | 4 K/mm$^3$ | 4.8 K/mm$^3$ |
| Red Blood Cells | 3 Mi/mm$^3$ | 7.2 Mi/mm$^3$ |
| Hemoglobin | 42 g/dl | 21 g/dl |
| Hematocrit | 46% | 64% |
| Platelet | 100 k/mm$^3$ | 120 k/mm$^3$ |

#### *Example 2 - Sterilization of Dextrose*

Dextrose (or glucose) containing solutions are used in the treatment of carbohydrate and fluid depletion, in the treatment of hypoglycemia, as a plasma expander, in renal dialysis and to counteract hepatotoxins (The Merck Index, Eleventh Edition, Merck & Co., Inc. (1989), and Martindale's Extra Pharmacopoeia, p.1, 265). Dextrose is also the preferred source of carbohydrate in parental nutrition regiments (The Merck Index, Eleventh Edition, Merck & Co., Inc. (1989), and Martindale's Extra Pharmacopecia, p.1, 265). In all of the above applications, the dextrose must be sterilized before use. Sterilization of dextrose-containing products is generally done by heat sterilization or autoclaving. Unfortunately, these methods have been reported to degrade or carmelize dextrose-containing solutions resulting in a color change in the solution (Martindale's Extra Pharmacopecia p.1, 265). Gamma irradiation of glucose has also been reported to decompose glucose-containing solutions (Kawakishi, et al., "Radiation-Induced Degradation of D-glucose in Anaerobic Contition," Agric. Biol. Chem., June 1977). Therefore, there is a need for a method that can sterilize dextrose-containing products that does not degrade the product itself. In view of the problems of the prior art, a dextrose solution was treated according to the method of the present invention as follows.

A 5% dextrose solution was irradiated for 24 hours, at a rate of approximately 1 kGy/hr. After irradiation, the product was tested and it was found that there was no visible light spectrum change as compared to the nonirradiated control. Therefore, the present method can be useful in sterilizing products that contain dextrose.

In addition to the above experiment, fresh solutions of 5% and 50% dextrose were irradiated to 25 kGy over 36 hours at ambient temperature. The results were similar to those described above. In addition, UV/VIS scans were obtained and demonstrated a complete absence of the peak at 283.4 nm for "furfural" as per U.S.P. In contrast, dextrose samples sterilized using an autoclave contain the 283.4 furfural peak. "Furfurals" are carcinogenic.

***Example 3 - Sterilization of Human Serum Albumin***

Normal Human Serum Albumin was irradiated as a 25% salt-poor solution to a total dose of 25 kGy over 36 hours using a Gammacell 220 ($Co^{60}$ is the gamma ray source in this instrument). The temperature was not controlled during the irradiation but it is estimated that the container holding the albumin solution was approximately 23°C. The results of HPLC analysis are given in Table 2.

TABLE 2

| Parameter | Control (%) | Irradiated (%) |
|---|---|---|
| Polymer | 2 | 3 |
| Dimer | 7 | 8 |
| Monomer | 90 | 86 |
| Low Molecular Weight | 1 | 3 |
| pH | 7.05 | 6.97 |
| NTU (must be > 20) | 11.4 | 11.4 |

As the results demonstrate, Normal Human Serum Albumin can safely be irradiated to 25 kGy (at a rate of approximately 0.7 kGy/hr) at room temperature without adversely affecting the essential properties of the protein. This has not been demonstrated before. All other attempts at irradiating serum albumin require that it be irradiated in the frozen stage. This adds to the cost and difficulty of doing the irradiation.

***Example 4***

Normal human blood from a healthy donor was taken in a heparinized tube, washed three times with standard CPD solution, then diluted 1:20 with CPD containing 0.01% v/v Ethanol. This latter solution of CPD with 0.01% v/v Ethanol is called SCPD. Two ml aliquots were then placed in 10 ml plastic test tubes and irradiated to different doses up to 26 kGy over 36 hours at room temperature. There was no haemolysis and the cells appeared intact if somewhat large and slightly irregular in shape. The results ofthree separate experiments are reported in Table 3.

TABLE 3

| Parameter | RCB[1] | HGB[2] | HCT[3] | MCV[4] | MCH[5] | MCHC[6] | RDW[7] | Flags |
|---|---|---|---|---|---|---|---|---|
| 1* | 1.08 | 41 | .097 | 89.5 | 38.3 | 427 | 17.7 | Nearly Normal |
| Control | .99 | 33 | 0.89 | 90.2 | 33.0 | 366 | 15.3 | |
| 2* | | | | 95.0 | 32.3 | 339 | 12.0 | |
| 12 kGy 1 | 1.22 | 45 | .166 | 135.8 | 36.5 | 269 | 27.3 | 1+Anisocytosis |
| | 1.38 | 45 | .199 | 144.7 | 33.0 | 228 | 24.9 | 3+Macrocytocis |
| 1 | 1.04 | 32 | .169 | 163.0 | 31.3 | 152 | 18.8 | 1+Anisocytosis |
| 16 kGy | 0.54 | 29 | .088 | 162.5 | 54.5 | 335 | 18.8 | 3+Macrocytocis |
| 2 | 0.82 | 27 | .128 | 156.5 | 32.8 | 209 | 19.8 | 2+Anisocytosis |
| | 0.81 | 26 | .124 | 152.6 | 32.4 | 212 | 20.2 | 3+Macrocytocis |
| 1 | 0.79 | 244 | .125 | 158.4 | 30.8 | 194 | 19.4 | 1+Anisocytosis |
| 20 kGy | 1.26 | 28 | .203 | 161.5 | 22.1 | 137 | 19.0 | 3+Macrocytocis |
| 2 | 0.93 | 30 | .141 | 151.5 | 32.3 | 213 | 20.1 | 2+Anisocytosis |

(continued)

| Parameter | RCB[1] | HGB[2] | HCT[3] | MCV[4] | MCH[5] | MCHC[6] | RDW[7] | Flags |
|---|---|---|---|---|---|---|---|---|
| 26 kGy 1 | 0.92 | 30 | .143 | 155.5 | 32.1 | 207 | 20.5 | 3+Macrocytocis |
| | 1.15 | 34 | .180 | 155.9 | 29.4 | 189 | 19.1 | 1+Anisocytosis |
| | 1.15 | 34 | .176 | 153.0 | 29.9 | 195 | 23.4 | 3+Macrocytocis |

* Experiment 1 and Experiment 2

1. Red Blood Cell Count: Cells x $10^{12}$/liter
2. Hemoglobin: grams/liter
3. Hematocrit
4. Mean Corpuscular Volume: Femtoliters
5. Mean Corpuscular Hemoglobin: picograms
6. Mean Corpuscular Hemoglobin Concentration: grams/liter

The cells were easily put into suspension and reconstituted in fresh buffer.

The following three experiments (Examples 5, 6 and 7) were conducted in order to determine the efficacy of the method when treating HIV-contaminated blood. In each Example the cells were similarly treated. In these experiments, the cells were gently agitated after 12, 16 and 24 hours of irradiation. Further, in the third experiment (Example 7), the cells were placed in T25 flasks to provide greater surface area and reduce the concentration due to settling in the bottom of the centrifuge tubes. In each case, the cells were irradiated at a dose rate of approximately 0.7 kGy/hr.

***Example 5 - Sterilization of HIV-containing Blood***

The following experiments were undertaken with the following specific objectives:

1. To evaluate the toxicity of the process towards red blood cells (RBCs).
2. To evaluate the anti-retroviral activity of the process.

***Method***

Initially, 2 ml of anticoagulated blood was obtained from an HIV-seronegative donor. The blood was centrifuged, and the plasma was removed. The remaining cell pellet was resuspended in 10 ml of the CPD buffer and centrifuged. This washing process was repeated a total of three times. The final pellet was resuspended in 40 ml of the SCPD buffer, and distributed into plastic tubes in 2 ml aliquots, with 16 separate aliquots being retained for further manipulation. For 8 of these tubes, an aliquote of HTLV-IIIB was added. This is a laboratory strain of the HIV virus and 100 tissue culture infective doses (TCID) were added to each of the tubes to be infected. For the remaining 8 tubes, a "mock" infection was performed, by adding a small amount of non-infectious laboratory buffer, phosphate buffered saline (PBS). Four infected and four non-infected tubes were subjected to the process. For comparison, the remaining 8 tubes (four infected and four non-infected) were handled in an identical manner, except that they were not subjected to the process.

It should be stated that at the beginning of the study, a separate aliquot of blood was obtained from the donor. This was processed in the clinical hematology laboratory and a complete hemogram was performed. These baseline results were compared to repeat testing on the study aliquots, which included evaluation of four processed and four unprocessed samples, all of which were not infected with HIV.

An aliquot of 0.5 ml of each of the infected study samples was inoculated on mononuclear cells (MCs) which had been obtained three days earlier. These cells had been suspended in RMPI culture medium, with 10% fetal calf serum and other additives (penicillin, streptomycin, glutamine and HEPES buffer) along with 1 $\mu$g/ml PHA-P. At the same time as this inocculation, the cells were resuspended in fresh medium with rIL-2 (20 U/ml). The cultures were maintained for 7 days. Twice weekly, a portion of the culture medium was harvested for the measurement of HIV p24 antigen levels (commercial ELISA kit, Coulter Electronics, Hialeah, FL) for the measurement of viral growth.

A separate aliquot of the eight infected study samples was used for viral titration experiments. Briefly, serial four-fold dilutions of the virus-containing fluids (ranging from 1:16 to 1:65,536) were inoculated in triplicate in 96-well flat-bottom tissue culture plates. PHA-stimulated MCs were added to each well (4 million cells in 2 ml culture medium, with IL-2). An aliquot of the supernatant from each culture well was harvested twice weekly for the measurement of HIV p24 antigen levels. A well was scored as "positive" if the HIV p24 antigen value was > 30 pg/ml.

The viral titer was calculated according to the Spearman-Karber method (se ACTG virology protocol manual) using the following equation:

$$M = xk + d[0.5 - (1/n)r]$$

M: titer (in log 4)
xk: dose of highest dilution
d: space between dilutions
n: number of wells per dilution
r: sum of total number of wells.

***Results***

Red blood cell parameters for the baseline sample as well asfor the unprocessed and processed study samples are shown in Table 4.

TABLE 4

| Sample / Number | MCV | MCH | MCHC |
|---|---|---|---|
| Baseline | 94.5 | 32.0 | 339 |
| Unprocessed-1 | 91.4 | 34.4 | 376 |
| Unprocessed-2 | 90.2 | 37.9 | 420 |
| Unprocessed-3 | 92.1 | 40.0 | 433 |
| Unprocessed-4 | 91.0 | 40.2 | 442 |
| Processed-1 | 133.4 | 37.8 | 284 |
| Processed-2 | 131.5 | 45.0 | 342 |
| Processed-3 | 128.5 | 38.9 | 303 |
| Processed-4 | 131.1 | 39.4 | 301 |

The abbreviations in Table 4 are explained under Table 3.
As described above, HIV cultures were established using 0.5 ml aliquots of unprocessed and processed study samples. P24 antigen levels (pg/ml) from the study samples on day 4 and day 7 of culture are shown in Table 5

TABLE 5

| Sample / Number | p24 Day 4 | p24 Day 7 |
|---|---|---|
| Unprocessed-1 | 1360 | 484 |
| Unprocessed-2 | 1180 | 418 |
| Unprocessed-3 | 1230 | 516 |
| Unprocessed-4 | 1080 | 563 |
| Processed-1 | 579 | 241 |
| Processed-2 | 760 | 303 |
| Processed-3 | 590 | 276 |
| Processed-4 | 622 | 203 |

Finally, one unprocessed sample and one processed sample were selected for the performance of direct viral titration without culture. The results are shown in Table 6.

TABLE 6

| Sample/Number | Titer (log 10 ml) |
|---|---|
| Unprocessed-1 | 1.5 |
| Processed-1 | 0.0 |

The red blood cells were minimally affected by the process, although some reproducible macrocytosis was observed. Although on co-culturing of processed samples, there appeared to be some residual live virus, this was not confirmed by direct titration experiments.

### *Example 6*

The objective of this experiment was to evaluate the toxicity of the proces towards red blood cells in a comprehensive manner.

### *Method*

For this experiment, 1 ml of anticoagulated blood was obtained from the same HIV-seronegative donor as in the first experiment. The blood was centrifuged and the plasma was removed. The remaining cell pellet was resuspended in 10 ml of the CPD buffer and centrifuged. This washing process was repeated a total of three times. The final pellet was resuspsnded in 20 ml of the SCPD buffer and distributed into plastic tubes in 2 ml aliquots with all 10 aliquots being retained for further manipulation. Eight tubes were subjected to the process, while the final two tubes were retained as control, unprocessed tubes. After the processing, all the tubes were centrifuged, and the resulting pellet was resuspended in 100 $\mu$l buffer. A complete hemogram was performed on these reconcentrated study samples.

As in the first experiment, a separate aliquot of blood was obtained from the donor when the study sample was taken. A complete hemogram was performed on this baseline sample. As the study samples were re-concentrated to 33-50% of their original state, more direct comparisons with the baseline sample could be undertaken than were possible in our earlier experiment.

### *Results*

Red blood cell parameters for the baseline sample as well asf or the unprocessed and processed study samples are shown in Table 7. The abbreviations used in Table 7 are defined in Table 3.

TABLE 7

| Sample / Number | RBC | HGS | MCV | MCH | MCHC |
|---|---|---|---|---|---|
| Baseline | 4.76 | 152 | 94.9 | 31.9 | 336 |
| Unprocessed-1 | 0.99 | 33 | 90.2 | 33.0 | 366 |
| Unprocessed-2 | 1.08 | 41 | 89.5 | 38.3 | 427 |
| Processed-1 | 1.15 | 34 | 153.0 | 29.9 | 195 |
| Processed-2 | 1.15 | 34 | 155.9 | 29.4 | 189 |
| Processed-3 | 1.26 | 28 | 161.5 | 22.1 | 137 |
| Processed-4 | 0.79 | 24 | 158.4 | 30.8 | 194 |
| Processed-5 | 0.54 | 29 | 162.5 | 54.5 | 335 |
| Processed-6 | 1.04 | 32 | 163.0 | 31.3 | 192 |
| Processed-7 | 1.35 | 45 | 144.7 | 33.0 | 228 |
| Processed-8 | 1.22 | 45 | 135.8 | 36.5 | 269 |

There was macrocytosis of the cells which was present in all the processed samples. Comparable hemoglobin levels were measured in the unprocessed and processed samples. The absolute values were appropriate for the residual dilution. The red blood cells are preserved.

### *Example 7*

### *Method*

For this experiment, 5 ml of anticoagulated blood was obtained from the same HIV-seronegative donor as in the first two experiments. The blood was centrifuged, and the plasma was removed. The remaining cell pellet was resuspended in 100 ml of the CPD buffer, and centrifuged. This washing process was repeated a total of three times. The final pellet was resuspended in 100 ml of the SCPD buffer and distributed in 25 ml aliquots, in T25 tissue culture flasks, with all four aliquots being retained for further manipulation. Two flakes were subject to the process, while the other two were retained as control, unprocessed flasks. After the processing, the contents of each of the flasks was observed and a visual determination of the cells' capacity to absorb oxygen (turning a brighter red on exposure to ambient air) was made. Following this, the contents of the flasks were aspirated and centrifuged, with the residual pallet resuspended in a small volume of buffer. A complete hemogram was performed on these reconcentrated study samples.

As in Examples 5 and 6, a separate aliquot of blood was obtained from the donor when the study sample was taken. A complete hemogram was performed on this baseline sample. As the study samples were re-concentrated to 33-50% of their original state, direct comparisons of a number of specific parameters would be possible with the baseline sample.

### *Results*

On visual inspection, there were no appreciable differences between the processed and unprocessed study samples. Specifically, there appeared to be a uniform distribution of well suspended cells. On exposure to ambient air, the contents of all flasks became somewhat brighter red. No specific quantitative measurements of oxygenation were made.

Red blood cell parameters for the baseline sample as well as for the unprocessed and processed study samples are shown in Table 8. The abbreviations used in Table 8 are defined under Table 3.

TABLE 8

| Sample / Number | RBC | HGS | MCV | MCH | MCHC |
|---|---|---|---|---|---|
| Baseline | 4.75 | 153 | 95.0 | 32.3 | 339 |
| Unprocessed-1 | 0.93 | 30 | 151.5 | 32.3 | 213 |
| Unprocessed-2 | 0.92 | 30 | 155.5 | 32.1 | 207 |
| Processed-1 | 0.82 | 27 | 156.5 | 32.8 | 209 |
| Processed-2 | 0.81 | 26 | 152.6 | 32.4 | 212 |

This experiment was designed to more closely approximate conditions of red blood cells to be transfused into a patient, and was consequently conducted at higher volumes. On a preliminary basis, it does not appear that the process impairs the red blood cells' ability to carry oxygen, although this should be measured more formally. Interestingly, in this experiment, there was no difference in cell size between the processed and unprocessed samples, both being large compared to baseline. Comparable hemoglobin levels were measured in all the study samples.

### *Example 8*

In this experiment, Immunoglobulin G (IgG) was irradiated in lyophilized form.

### *Method*

The results of HPLC analysis of IgG are given in Table 9. AS the results demonstrate, the product appears to be unaffected after being irradiated to a dose of 25 kGy at room temperature when the irradiation is delivered at a rate of approximately 0.7 kGy/hr. This has not been previously demonstrated.

TABLE 9

| Parameter | Control (%) | Irradiated (%) |
|---|---|---|
| Polymer (must be >2%) | 1 | 1 |
| Dimer | 10 | 13 |
| Monomer | 88 | 84 |
| Low Molecular Weight | 1 | 2 |

### *Results*

The results presented by Gergely, *et al.,* using freeze dried IgG showed that a portion of the protein was insoluble after an irradiation dose of 12 kGy to 25 kGy at standard irradiation dose rates. (Gergely, J., et al., "Studies of Gama-Ray-Irradiated Human Immunoglobulin G." SM-92/12 I.A.E.A.) In contrast, using the present method at a dose rate of approximately .7 kGy/hr, none of the protein was insoluble. This would indicate that little or no change or degradation of the protein occurred. Further, Gergely, et al., found that a liquid formulation of human IgG lost all of its activity after irradiation. In studies using the present method on intravenous immunoglobulin (IVIG) in liquid form, it was shown that greater than 70% of a specific antibody in hyperimmune IVIG was retained.

***Example 9***

In this experiment, alpha I proteinase inhibitor and fibrinogen were irradiated in lyophilized form.

***Method***

The samples were placed in a Gammacell 220 and irradiated according to the present process to a total dose of 25 kGy. Samples were then returned to the laboratory for analysis. The dose rate was 0.72 kGy/hr.

***Results***

The alpha 1 proteinase inhibitor, both treated and control, were 40% of a standard normal pooled plasma sample. The Mancini radial immunodiffusion technique was used as the assay.

The topical fibrinogen complex vials were reconstituted in 10 ml of water. Protamine sulphate vials were reconstituted in 10 ml of water. Protamine sulphate at a concentration of 10 mg/ml was added to the samples. There was instant formation of monomer in all three preparations.

***Example 10***

In this experiment, Factors VII, VIII and IV were irradiated in lyophilized form.

***Method***

The samples were placed in a Gamacell 220 and irradiated to various total doses at a dose rate of approximately 1 kGy/hr.

***Results***

Factor VII retained 67% activity at 20 kGy and 75% at 10 kGy. Factor VIII retained 77% activity at 20 kGy and 88% at 10 kGy. Similarly, Factor IV showed an activity level of 70% at 20 kGy and 80% at 10 kGy.

Excellent results were found for the three Factors. To our knowledge, no one has been able to achieve these results by irradiating the Factors at ambient temperature to such a high dose of radiation with such little loss of activity. This is in direct contrast with the results of Kitchen, et al., "Effect of Gamma Irradiation on the Human Immunodeficiency Virus and Human Coagulation Proteins," Vox Sang 56:223-229 (1989), who found that "the irradiation of lyophilized concentrates is not a viable procedure." Similarly, Hiemstra, et al., "Inactivation of human immunodeficiency virus by gamma radiation and its effect on plasma and coagulation factors," Transfusion 31 :32-39 (1991), also concluded that "Gamma radiation must be disregarded as a method for the sterilization of plasma and plasma-derived products, because of the low reduction of virus infectivity at radiation doses that still give acceptable recovery of biologic activity of plasma components."

***Example 11***

In this experiment, red blood cells were irradiated at a dose rate of 0.5 kGy/hr for periods of time ranging from 7.5 to 90 minutes in order to remove bacterial contaminants.

***Method***

Red blood cells were collected from a healthy donor in EDTA, washed 3 times with CPD solution and resuspended in DPC to provide a 1:20 dilution based on the original blood volume. The cell suspension was then subdivdied into 14 tubes. To seven of the tubes, approximately 1.0 x $10^4$ Staphylococcus epidermidia were added. The cells were placed on ice for transport to the irradiation facility. All of the samples were placed in the chamber at ambient temperature and irradiated at 0.5 kGy/hr for periods of time to give total doses of 0.625, 0.125, 0.250, 0.375, 0.500 and 0.750 kGy, respectively. The samples were removed and agitated at each time point and placed on ice for transport either to the microbiology lab or the hematology lab for analysis.

***Results***

The results of the microbiology assays are given in Table 10.

TABLE 10

| Radiation Dose (kGy) | Time (Min.) | Number Surviving |
|---|---|---|
| 0 | | 92,200 |
| 0.625 | 7.5 | 84,500 |
| 0.125 | 15 | 35,000 |
| 0.250 | 30 | 10,067 |
| 0.375 | 45 | 1,800 |
| 0.500 | 60 | 250 |
| 0.750 | 90 | 0 |

Thus, a dose of 0.75 kGy provides a 4.5 $\log_{10}$ reduction in bacterial survivors. This represents a significant safety factor for blood. Further, the D 10 value is approximately 0.125 kGy which corresponds well with the values reported in the literature for similar species of staphylococcus (B.A. Bridges, "The effect of N-Ethylmaleimide on the radiation sensitivity of bacteria," J. Gen. Microbiol. 26:467-472 (1962), and Jacobs, G.P. and Sadeh, N., "Radiosensitization of Staphyloccocus aureus by p-hydroxybenzoic acid," Int. J. Radiat. Biol. 41:351-356 (1982).

In order to demonstrate that the red blood cells remained viable after the irradiation process, the following parameters were determined for the cells, WBC, Neutrophils, Lymphocytes, Monocytes, Eosinophils and Basophils. These determinations merely enumerated the number of cells present. All nucleated cells would, of course, be inactivated by the radiation dose delivered. The other red blood cell parameters monitored are listed in Table 11. The Methaemoglobin value was unchanged from that of the controls even after a radiation dose of 0.75 kGy. This experiment demonstrates that red blood cells can be safely irradiated by the present method to a dose of 0.75 kGy at room temperature with no loss of cell function.

***Example 12***

This experiment was conducted using the method in Example 11 to confirm the findings of Example 11 and to expand upon some of the parameters measured. The results of this experiment are given in Table 12.

***Results***

(See Table 12, below.)

These results confirm the previous results and indicate that indeed, red blood cells can be irradiated to a dose sufficient to provide 4.5 $\log_{10}$ reduction in bacterial count.

It is contemplated that future experiments will provide similar results for platelet. Thus, with little or no additional manipulation, and without the addition of extraneous materials, red blood cells can be treated by the present process to provide a bacteriologically safe product, thus further reducing the risk of untoward reactions in recipients.

TABLE 11

Red Blood Cell Valus as a Function of Radiation Dose Received

| | Total Dose (in kGy) | | | | | |
|---|---|---|---|---|---|---|
| **Parameter** | **Whole Blood** | **0** | **0.625** | **0.125** | **0.250** | **0.500** |
| **RBC** | 5.06 | 1.49 | 1.27 | 1.77 | 1.73 | 1.43 |
| **HGB** | 153 | 43 | 41 | 56 | 56 | 46 |
| **HTC** | .483 | .142 | .120 | .156 | .163 | 1.31 |
| **MCV** | 95.5 | 95.6 | 94.3 | 94.2 | 93.7 | 32.1 |
| **MCH** | 31.2 | 31.1 | 32.2 | 31.7 | 32.2 | 32.5 |
| **MCHC** | 327 | 325 | 341 | 336 | 344 | 353 |
| **RDW** | 13.93 | 12.1 | 12.7 | 12.9 | 12.9 | 13.2 |
| **METHgB** | 0.9 | 0.3 | 0.3 | 0.3 | 0.0 | 0.9 |

TABLE 12 Red Blood Cell Valus as a Function of Radiation Dose Received

| | Total Dose (in kGy) | | | | | | |
|---|---|---|---|---|---|---|---|
| **Parameter** | **0** | **0.625** | **0.125** | **0.250** | **0.375** | **0.555** | **0.750** |
| **HGB** | 1.8 | 1.7 | 1.8 | 1.7 | 2.0 | 2.0 | 2.0 |
| **% O** | 96.6 | 96.5 | 96.2 | 96.3 | 96.4 | 96.5 | 96.0 |
| **% CO** | 1.0 | 1.2 | 1.6 | 1.3 | 1.7 | 1.5 | 1.5 |
| **% NET** | 0.5 | 0.5 | -0.5 | 0.4 | -0.2 | 0.4 | 0.8 |
| **% Reduced** | 1.9 | 1.9 | 2.7 | 2.4 | 3.2 | 1.7 | 1.7 |
| **p60 (mm Hg)** | 34 | nd | nd | nd | nd | nd | 24 |
| **Hill Coefficient** | 2.1 | nd | nd | nd | nd | nd | 1.8 |

nd = not done

The uncertainty with the methaemoglobin levels is ±2%; with the p50 it is ±4% (95% confidence).

***Example 13***

In this experiment, the protective effects of certain stabilizers were evaluated using lyophilized anti-insulin monoclonal antibody exposed to 45 kGy of low dose gamma irradiation. The stabilizers tested were: sodium ascorbate, methionine, and lipoic acid.

***Method***

In 2 ml glass vials, 0.5 ml total volume was lyophilized containing 50μg anti-insulin monoclonal anti-body, 5 mg bovine serum albumin (1%) and either no stabilizer or 50mM of the stabilizer of interest. The samples were stoppered under vacuum. Samples were irradiated with gamma radiation (45 kGy total dose, dose rate 1.83 kGy/hr, temperature 4°C) and then reconstituted with water.

Antibody binding activity of independent duplicate samples was determined by a standard ELISA protocol: 96-well microtitre plates were coated overnight with 2.5μg/ml insulin antigen. Three-fold serial dilutions of anti-insulin monoclonal antibody samples starting at 5μg/ml were used. Goat anti-mouse Ig conjungated to phosphatase used at 50 ng/ml. Sigma 104 alkaline phosphatase substrate was used at 1 mg/ml in DEA buffer. Binding activity was determined by absorbance at 405-620nm.

Relative protection was determined by estimating the shift in the titration curve (*i.e.* concentration of antibody needed to observe the same amount of binding) of the irradiated sample compared to an unirradiated sample at approximately 50% of the maximum absorbance signal for the unirradiated sample.

***Results***

Lyophilized samples containing no stabilizer retained 50% of antibody avidity following irradiation with 45 kGy gamma irradiation. This is in contrast to previous results in which 45 kGy of gamma radiation destroyed essentially all the activity of immunoglubulin when it was irrradiated in solution. Thus, it is apparent that the reduction in residual water content by lyophilizing afforded significant protection on its own protein.

The addition of sodium ascorbate provided full recovery of activity after irradiation of the sample. Both methionine and lipoic acid provided significant recovery of activity (76-83%) of activity after irradiation as compared to the unirradiated sample. The results are shown in Figures 1 and 2.

***Example 14***

In this experiment, the protective effects of certain stabilizers were evaluated using lyophilized anti-insulin monoclonal antibody exposed to 45 kGy of low dose gamma irradiation. The stabilizers tested were: sodium ascorbate, N-acetyl cysteine, glutathione and mixtures of urate/trolox and ascorbate/urate/trolox.

### *Method*

In 3 ml glass vials, 1.0 ml total volume was lyophilized containing 100μg anti-insulin monoclonal anti-body, 10 mg bovine serum albumin (1%) and either no stabilizer or the stabilizer of interest. The samples were stoppered under vacuum. Samples were irradiated with gamma radiation (45 kGy total dose, dose rate 1.83 kGy/hr, temperature 4°C) and then reconstituted with 1.0 ml water.

Antibody binding activity of independent duplicate samples was determined by a standard ELISA protocol: Maxisorb plates were coated overnight with 2.5μg/ml insulin antigen. Three-fold serial dilutions of anti-insulin mAb samples starting at 5μg/ml were used. Goat anti-mouse Ig conjugated to phosphatase was used at 50 ng/ml. Binding activity was determined by absorbance at 405-620nm.

Relative protection was determined using a parallel line analysis software package (PLA 1.2 from Stegmann Systemberatung).

### *Results*

Lyophilized samples containing no stabilizer retained 70% of antibody avidity following irradiation with 45 kGy gamma irradiation. This is in contrast to previous results in which 45 kGy of gamma radiation destroyed essentially all the activity of immunoglubulin when it was irrradiated in solution. Thus, it is apparent that the reduction in residual water content by lyophilizing afforded significant protection on its own protein.

The presence of sodium ascorbate increased recovery by 20%, *i.e.* such that there is 90% avidity recovered after irradiation. The remaining stabilizers resulted in recovery of 77-84% of avidity. The results are shown in Figures 3A-3C.

### *Example 15*

In this experiment, the protective effects of primary lyophilizing (which leaves a relatively "high moisture" content in the product) and secondary lyophilizing (which results in a product with relatively "low moisture") on the sensitivity of a monoclonal antibody were determined.

### *Methods*

In 3 ml glass vials, 1.0 ml total volume was lyophilized containing 100μg anti-insulin monoclonal anti-body, 10 mg bovine serum albumin (1%) and either no stabilizer or 100 mM of sodium ascorbate. The samples were stoppered under vacuum. Samples were irradiated with gamma radiation (45 kGy total dose, dose rate between 2.03 and 2.13 kGy/hr, temperature 4°C) and then reconstituted with 1.0 ml water.

Antibody binding activity of independent duplicate samples was determined by a standard ELISA protocol: Maxisorb plates were coated overnight with 2.5μg/ml insulin antigen. Three-fold serial dilutions of anti-insulin mAb samples starting at 5μg/ml were used. Goat anti-mouse Ig conjugated to phosphatase was used at 50 ng/ml. Binding activity was determined by absorbance at 405-620nm.

### *Results*

In the absence of a stabilizer, there was better recovery of the anti-insulin mAb after irradiation from the samples that had undergone the secondary "low moisture" drying cycle, i.e. a lower total moisture content in the absence of a stabilizer improved recovery.

In the presence of the stabilizer, however, there was very good recovery of antibody activity after 45 kGy irradiation, irrespective of whether the sample had undergone only the primary "high moisture" drying cycle or had also undergone the secondary "low moisture" drying cycle.

The results of this experiment are shown in Figure 4.

### *Example 16*

In this experiment, the protective effect of lyophilizing and/or an added stabilizer on the activity of Factor VIII was determined. The stabilizers tested were; sodium ascorbate; sodium urate; trolox; ascorbate/trolox mixtures; ascorbate/urate/trolox mixtures; urate/trolox mixtures; ascorbate/urate mixtures

*Methods*

Samples were lyophilized and stoppered under vacuum. Samples were irradiated with gamma radiation (45 kGy total dose, dose rate 1.9 kGy/hr, temperature 4°C) and then reconstituted with water. Measurement of Factor VIII activity in the samples was determined in a one-stage clotting assay using an MLA Electra 1400C Automatic Coagulation Analyzer.

*Results*

In the absence of a stabilizer, there was good recovery of Factor VIII activity after irradiation of the lyophilized sample (69-88% of unirradiated control). In the presence of a stabilizer, there was similar recovery of Factor VIII activity after irradiation (69-89% of unirradiated control).

The combination of a stabilizer and lyophilizing, however, provided a recovery of Factor VIII of between 83-90% of the unirradiated control (sodium ascorbate + lyophilizing: 90% recovery; trolox + lyophilizing: 84% recovery; and sodium urate + lyophilizing: 83%). The results are shown in Figure 5.

*Example 17*

In this experiment, the protective effects of certain stabilizers were evaluated using liquid or lyophilized antithrombin III (ATIII) exposed to 25 kGy of low dose gamma irradiation. The stabilizer tested was sodium ascorbate (200 mM).

*Method*

ATIII was either irradiated alone or in the presence of ascorbate as a stabilizer. Mixing with the stabilizer was accomplished by either: (i) mixing the ATIII and the stabilizer as liquids and then lyophilizing the mixture and stoppering under vacuum; or (ii) mixing the ATIII and the stabilizer while both were dry powders (*i.e.* after each was lyophilized separately).

After irradiation (25 kGy total dose, 1.8 kGy/hr rate), the lyophilized powder antithrombin III (Sigma A 9141, lot 113H9316) + ascorbate was reconstituted to a concentration of 40U/ml with water. Following irradiation, both the liquid and reconstituted dry powder AT III samples (+ ascorbate) were then diluted to 20 U/ml in water. Thrombin (1U/ml) and heparin (800 U/ml) solutions in water were prepared.

In a pre-chilled 96-well plate assay, 2-fold serial dilutions of the AT III samples were prepared. Heparin (25μl of 800 U/ml solution) or water was added to each well, followed by incubation at 37°C. Thrombin (50μl of 1U/ml solution) was added, again followed by incubation at 37°C.

100μl of 1600μM thrombin substrate in water was then added (final concentration of substrate was 800μM), followed by incubation at ambient temperature. Activity was determined by measuring absorbance between 405-620nm at fixed times following substrate addition.

*Results*

Liquid AT III lost all thrombin inhibitory activity in the absence of a stabilizer when irradiated at 25 kGy of low rate gamma irradiation. The presence of sodium ascorbate, however, maintained 55-66% of liquid AT III activity following irradiation.

Dry powder AT III lost only 43% of activity in the presence of a dry powder stabilizer when irradiated at 25 kGy of low dose gamma irradiation.

The results of this experiment are shown in Figure 6.

*Example 18*

In this experiment, the protective effect of certain stabilizers on the activity of lyophilized anti-insulin monoclonal antibody was determined. The stabilizers tested were; sodium ascorbate; trolox/urate/ascorbate mixtures; N-acetyl cysteine and glutathione.

*Methods*

Anti-insulin monoclonal antibody supplemented with 1 % of human serum albumin (and, optionally, 5% sucrose) was lyophilized, stoppered under vacuum, and irradiated (total dose 45 kGy; dose rate between 1.83 and 1.88 kGy/hr).

Antibody binding activity was determined using the standard ELISA protocol described above.

### *Results*

Irradiation of lyophilized anti-insulin mAb supplemented with 1% HSA to a dose of 45 kGy resulted in an average loss of avidity of about 33%. The addition of the following stabilizers significantly improved recovery: 20mM sodium ascorbate (100% recovery); 200μM trolox/1.5mM urate/20 mM ascorbate (87%) recovery); 20 mM N-acetyl cysteine (82% recovery) and 20 mM glutathione (76% recovery).

The addition of 5% sucrose to the lyophilized mAb containing 1% HSA resulted in an average loss of avidity of about 30% when irradiated to a dose of 45 kGy. The addition of the following stabilizers significantly improved recovery: 20mM sodium ascorbate (88% recovery); 200μM trolox/1.5mM urate/20 mM ascorbate (84%) recovery); 20 mM N-acetyl cysteine (72% recovery) and 20 mM glutathione (69% recovery).

The results of these experiments are shown in Figures 7-14.

### *Example 19*

In this experiment, the protective effect of stabilizers (ascorbate) on the activity of lyophilized anti-insulin monoclonal antibody was determined when the sample was irradiated at a high dose rate (30 kGy/hr).

### *Methods*

Anti-insulin monoclonal antibody was lyophilized and irradiated at a rate of 30 kGy/hr (total dose 45 kGy). Antibody binding activity was determined using the standard ELISA protocol described above.

### *Results*

Irradiation of lyophilized anti-insulin mAb to a dose of 45 kGy resulted in an average loss of activity of about 32%. The addition of 20mM sodium ascorbate provided 85% recovery of avidity compared to an unirradiated sample. The results are shown in Figure 15.

### *Example 20*

In this experiment, lyophilized thrombin was irradiated in the presence of a stabilizer.

### *Method*

Low dose rate samples were gamma irradiated at ambient temperature at a dose rate of 0.326 kGy/hr for a total dose of 45 kGy. High dose rate samples were gamma irradiated at ambient temperature at a dose rate of 30 kGY/hr for a total dose of 45 kGy.

Following irradiation, all samples were reconstituted with 500 μl of 50% glycerol solution to a concentration of 100 U/ml and then diluted to 0.5 U/ml. Thrombin activity was then determined by a standard chromogenic assay utilizing a SAR-Pro-Arg-PNA substrate.

Thrombin Vmax and Km values were determined by Sigma Plot 2000 using the singular rectangular hyperbolic fit equation for each averaged set of data. Thrombin activity was also determined using a clotting time assay performed on an MLA 1400C analyzer.

### *Results*

The calculated Vmax from thrombin irradiated at 30 kGy/hr at ambient temperature was 0.216, as compared to a Vmax of 0.287 for its unirradiated control, indicating a 77% recovery of thrombin activity.

The calculated Vmax from thrombin irradiated at 0.326 kGy/hr at ambient temperature was 0.189, as compared to a Vmax of 0.264 for the unirradiated control, indicating a 72% recovery of thrombin activity. A clotting time assay performed on the low dose sample yielded a 74% relative potency compared to the unirradiated control.

The results of this experiment are shown in Figure 16.

### *Example 21*

In this experiment, an IgM monoclonal antibody specific for murine $IgG_3$ was irradiated at a low dose rate in the presence or absence of a stabilizer.

***Method***

Liquid rat anti-murine $IgG_3$ monoclonal IgM antibody (in a PBS buffer with 10 mM sodium azide; concentration of antibody was 666 ng/μl) was irradiated at a rate of 1.8 kGy/hr to a total dose of either 10 kGy or 45 kGy. Samples either contained no stabilizer or a stabilizer mixture containing 20 mM citrate, 300 μM urate and 200 mM ascorbate.
Antibody activity was analyzed by standard ELISA protocol using murine IgG3 as the coating antigen and a phosphatase-conjugated anti-rat IgM detection antibody.

***Results***

Liquid samples containing no stabilizer lost all functional antibody activity following irradiation with either 10kGy or 45 kGy gamma irradiation. The presence of a stabilizer mixture, however, provided full recovery of activity following irradiation with 10 kGy gamma radiation and 88% recovery of activity following irradiation with 45 kGy gamma radiation. The results of this experiment are shown graphically in Figure 17.

***Example 22***

In this experiment, lyophilized and liquid samples of albumin were irradiated with gamma irradiation.

***Method***

Samples were irradiated with a total dose of either 10 kGy or 40 kGy gamma radiation. Following irradiation, the lyophilized samples were reconstituted with 1.1 ml of assay buffer (50 mM Tris, pH 8.8; 50 mM NaCl; 0.1 % PEG 8000). Samples (lyophilized and liquid) were analyzed by size-exclusion column chromatography (TSKgel G4000SWxl 30 cm x 7.8 mm; elution buffer 0.1 M sodium phosphate pH 6.5/0.1 M sodium sulfate; flow rate 1 ml/min) with a UV detection system set at 280nm.

***Results***

No degradation products were observed in the liquid or lyophilized samples of albumin irradiated with a total dose of 10 kGy gamma radiation. Although some degradation product was observed in the liquid samples of albumin irradiated with a total dose of 40 kGy gamma radiation, no such degradation was observed in the lyophilized samples irradiated with a total dose of 40 kGy gamma radiation. The chromatographic results of this experiment are shown in Figure 18.

***Experiment 23***

This experiment measured the sensitivity of prions (transmissible spongiform encephalopathy agents) to ionizing radiation at low dose rates.

***Method***

0.3 ml of phosphate buffered saline containing a 10% homogenate (brains collected from golden Syrian hamsters in the terminal stages of scrapie infection) was added to 29.7 ml of albumin in a 50 ml polypropylene tube. Samples were irradiated with a total dose of either 30 kGy or 55 kGy (control samples were not irradiated).
Weanling golden Syrian hamsters were inoculated intracerebrally with 50 μl of undiluted sample. All animals were then evaluated for signs of scrapie disease and scored for the appearance of a wobbling gait, failure to rear and terminal condition (at which point they were euthanized). The days post inoculation for each sign for each animal was calculated.

***Results***

Irradiation at the higher total dose (55 kGy) provided a thirteen to fifteen day delay in the median incubation times compared to the unirradiated control for any of the three symptomatic endpoints, which is equivalent to an approximately 2 $log_{10}ID_{50}$ reduction in the titer of the pathogen. Irradiation at the lower total dose (30 kGy) provided an eight to thirteen day delay in incubation time, which is equivalent to an approximately 1 $log_{10}ID_{50}$ reduction in the titer of the pathogen. This was still significantly loner than the unirradiated control.

Linear regression analysis of the data results in 95% confidence intervals that indicate that the actual reduction in pathogen levels may be as high as a 3.5 $log_{10}ID_{50}$ reduction in the titer of the pathogen.

***Experiment 24***

This experiment evaluated the protective effect of lyophilizing and/or the presence of a stabilizer on thrombin activity following irradiation with 45 kGy gamma radiation.

***Method***

Samples of thrombin were prepared containing 1 % bovine serum albumin and lyophilized to the desired level of moisture. Sodium ascorbate was added to a concentration of 200 mM in some samples as a stabilizer.
All samples were then irradiated with gamma radiation (45 kGy total dose, rate 2.03 to 2.13 kGy/hr at 4°C) and thrombin activity measured.

***Results***

In the absence of stabilizer, 67% of thrombin activity was recovered from the irradiated samples that had undergone only a primary drying cycle. The addition of a stabilizer increased the recovery to 86%. The results of this experiment are shown graphically in Figure 19.

***Example 25***

In this experiment, the protective effects of certain stabilizers were evaluated using liquid IVIG polyclonal antibody exposed to 45 kGy of gamma irradiation (1.8 kGy/hr). The stabilizers tested were: sodium ascorbate and a mixture of sodium ascorbate and N-acetyl cysteine.

***Results***

Irradiated samples containing no stabilizer exhibited the following losses in activity: 1 log with respect to rubella; 0.5-0.75 log with respect to mumps; and 1 log with respect to CMV. Irradiated samples containing sodium ascorbate or a mixture of sodium ascorbate and N-acetyl cysteine as a stabilizer exhibited no loss in activity when compared to unirradiated controls.
The results of this experiment are shown graphically in Figures 20-26.

***Example 26***

This experiment was designed to examine the effects of pH on the recovery of urokinase (liquid or lyophilized) irradiated in the presence of a stabilizer (sodium ascorbate, sodium urate or a mixture thereof).

***Method***

Urokinase (1000 U/ml) was mixed with 200 mM sodium ascorbate and/or 300 μM in the presence of 35 mM phosphate buffer at various pHs. All tested samples were irradiated with a total dose of 45 kGy gamma radiation at a rate of 2 kGy/hr.

***Results***

The lyophilized irradiated samples containing sodium ascorbate exhibited a recovery of about 88-90% of urokinase activity across the pH range of 5.5-7.8, inclusive. The liquid irradiated samples containing sodium ascorbate exhibited a recovery of about 65-70% of urokinase activity across the pH range 5.5-7.8. The results of this experiment are shown graphically in Figure 26.
Having now fully described this invention, it will be understood to those of ordinary skill in the art that the methods of the present invention can be carried out with a wide and equivalent range of conditions, formulations, and other parameters without departing from the scope of the invention or any embodiments thereof. All patents and publications cited herein are hereby fully incorporated by reference in their entirety.

Some additional embodiments of the invention are described in the following paragraphs.

1. A method for sterilizing a biological material that is sensitive to ionizing radiation, said method comprising:

(i) reducing the residual solvent content of a biological material to a level effective to protect said biological material from said ionizing radiation; and
(ii) irradiating said biological material with a suitable ionizing radiation at an effective rate for a time effective to sterilize said biological material.

2. A method for sterilizing a biological material that is sensitive to ionizing radiation, said method comprising:

(i) adding to a biological material at least one stabilizer in an amount effective to protect said biological material from said ionizing radiation; and
(ii) irradiating said biological material with a suitable ionizing radiation at an effective rate for a time effective to sterilize said biological material.

3. A method for sterilizing a biological material that is sensitive to ionizing radiation, said method comprising:

(i) adding to a biological material at least one stabilizer in an amount effective to protect said biological material from said ionizing radiation; and
(ii) irradiating said biological material with a suitable ionizing radiation at a low rate for a time effective to sterilize said biological material.

4. A method for sterilizing a biological material that is sensitive to ionizing radiation, said method comprising:

(i) reducing the residual solvent content of a biological material to a level effective to protect said biological material from said ionizing radiation; and
(ii) irradiating said biological material with a suitable ionizing radiation at a low rate for a time effective to sterilize said biological material.

5. A method for sterilizing a biological material that is sensitive to ionizing radiation, said method comprising:

(i) reducing the residual solvent content of a biological material to a level effective to protect said biological material from said ionizing radiation;
(ii) adding to said biological material at least one stabilizer in an amount effective to protect said biological material from said ionizing radiation; and
(iii) irradiating said biological material with a suitable ionizing radiation at an effective rate for a time effective to sterilize said biological material,

wherein steps (i) and (ii) may be performed in inverse order.

6. The method according to paragraph 2 or 3, further comprising the step of reducing the residual solvent content of said biological material prior to said step of irradiating said biological material.

7. The method according to paragraph 1, 4, 5 or 6, wherein said solvent is water.

8. The method according to paragraph 1, 4, 5 or 6, wherein said solvent is an organic solvent.

9. The method according to paragraph 7, wherein said residual water content is reduced by the addition of an organic solvent.

10. The method according to paragraphs 1-10, wherein said biological material is suspended in an organic solvent following reduction of said residual solvent content.

11. The method according to paragraphs 1-10, wherein said biological material is blood or a component of blood.

12. The method according to paragraphs 1-11, wherein said biological material is a proteinaceous material.

13. The method according to paragraph 12, wherein said proteinaceous material is a component of blood.

14. The method according to paragraphs 1-13, wherein said biological material is a clotting factor.

15. The method according to paragraph 14, wherein said clotting factor is selected from the group consisting of: Thrombin, Factor II, Factor V, Factor VII, Factor VIIa, Factor VIII, Factor IX, Factor X, Factor XIII, Factor XIIIa, Von Willebrand's Factor and Fibrinogen.

16. The method according to paragraphs 1-10, wherein said biological material is selected from the group consisting of: albumin, urokinase, polyclonal immunoglobulins, monoclonal immunoglobulins, and mixtures of one or more polyclonal and/or monoclonal immunoglobulins.

17. The method according to paragraph 16, wherein said immunoglobulins are immunoglobulin G, immunoglobulin M, or mixtures thereof.

18. The method according to paragraphs 1-10, wherein said biological material is mammalian tissue or a component of or processed mammalian tissue.

19. The method according to paragraphs 1-10, wherein said biological material is bone or a component of or processed bone.

20. The method according to paragraph 19, wherein said biological material is demineralized bone matrix.

21. The method according to paragraphs 1-10, wherein said biological material is a recombinantly-produced biological material.

22. The method according to paragraphs 1-10, wherein said biological material is a transgenic biological material.

23. The method according to paragraphs 1-10, wherein said biological material is a food or a botanical product.

24. The method according to paragraphs 1-10, wherein said biological material is a carbohydrate or polysaccharide.

25. The method according to paragraphs 1-10, wherein said biological material is selected from the group consisting of chitin, chitosan, NOCC-chitosan and derivatives thereof.

26. The method according to paragraphs 1-10, wherein said biological material is a product of cellular metabolism.

27. The method according to paragraphs 1-26, wherein said effective rate is not more than about 3.0 kGy/hour.

28. The method according to paragraphs 1-26, wherein said effective rate is not more than about 2.0 kGy/hr.

29. The method according to paragraphs 1-26, wherein said effective rate is not more than about 1.0 kGy/hr.

30. The method according to paragraphs 1-26, wherein said effective rate is not more than about 0.3 kGy/hr.

31. The method according to paragraphs 1, 2, 5-26, wherein said effective rate is more than about 3.0 kGy/hour.

32. The method according to paragraphs 1, 2, 5-26, wherein said effective rate is at least about 6.0 kGy/hour.

33. The method according to paragraphs 1, 2, 5-26, wherein said effective rate is at least about 18.0 kGy/hour.

34. The method according to paragraphs 1, 2, 5-26, wherein said effective rate is at least about 30.0 kGy/hour.

35. The method according to paragraphs 1-34, wherein said biological material is maintained in a low oxygen atmosphere..

36. The method according to paragraph 35, wherein said biological material is maintained in an argon atmosphere.

37. The method according to paragraphs 1, 4-36, wherein said residual solvent content is reduced by lyophilization.

38. The method according to paragraph 1, 4-37, wherein said residual solvent content is less than about 10.0%.

39. The method according to paragraph 1, 4-37, wherein said residual solvent content is less than about 5.0%.

40. The method according to paragraph 1, 4-37, wherein said residual solvent content is less than about 2.0%.

41. The method according to paragraph 1, 4-37, wherein said residual solvent content is less than about 1.0%.

42. The method according to paragraphs 1, 4-37, wherein said residual solvent content is less than about 0.5%.

43. The method according to paragraphs 1-42, wherein at least one sensitizer is added to said biological material prior to said step of irradiating said biological material.

44. The method according to paragraphs 1-43, wherein said biological material contains at least one prion as a biological contaminant.

45. The method according to paragraphs 1-43, wherein said biological material contains at least one virus as a biological contaminant.

46. The method according to paragraphs 1 and 4, wherein at least one stabilizer is added to said biological material prior to said step of irradiating said biological material.

47. The method according to paragraphs 2, 3, 5-46, wherein said at least one stabilizer is an antioxidant.

48. The method according to paragraphs 2, 3, 5-47, wherein said at least one stabilizer is a free radical scavenger.

49. The method according to paragraphs 2, 3, 5-47, wherein said at least one stabilizer reduces damage due to reactive oxygen species.

50. The method according to paragraphs 2, 3, 5-47, wherein said at least one stabilizer is selected from the group consisting of: ascorbic acid or a salt or ester thereof; glutathione; 6-hydroxy-2, 5, 7, 8-tetramethylchroman-2-carboxylic acid; uric acid or a salt or ester thereof; methionine; histidine; N-acetyl cysteine; and mixtures of two or more of said stabilizers.

51. The method according to paragraph 50, wherein said mixtures of two or more of said stabilizers is selected from the group consisting of: mixtures of ascorbic acid, or a salt or ester thereof, and uric acid, or a salt or ester thereof; mixtures of ascorbic acid, or a salt or ester thereof, and 6-hydroxy-2, 5, 7, 8-tetramethylchroman-2-carboxylic acid; mixtures of ascorbic acid, or a salt or ester thereof, uric acid, or a salt or ester thereof, and 6-hydroxy-2, 5, 7, 8 tetramethylchroman-2-carboxylic acid; and mixtures of uric acid, or a salt or ester thereof and 6-hydroxy-2, 5, 7, 8-tetramethylchroman-2-carboxylic acid.

52. The method according to paragraphs 1-51, wherein said ionizing radiation is corpuscular radiation or electromagnetic radiation or a mixture thereof.

53. The method according to paragraph 52, wherein said electromagnetic radiation is selected from the group consisting of radio waves, visible and invisible light, ultraviolet light, x-ray radiation, and gamma radiation.

54. The method according to paragraphs 1-51, wherein said ionizing radiation is gamma radiation.

55. The method according to paragraphs 1-51, wherein said ionizing radiation is e-beam radiation.

56. The method according to paragraphs 1-51, wherein said ionizing radiation is visible light.

57. The method according to paragraphs 1-51, wherein said ionizing radiation is ultraviolet light.

58. The method according to paragraphs 1-51, wherein said ionizing radiation is x-ray radiation.

## Claims

**1.** A method for sterilizing a biological material that is sensitive to ionizing radiation, said method comprising:

(i) adding to a biological material at least one stabilizer in an amount effective to protect said biological material from said ionizing radiation; and
(ii) irradiating said biological material with a suitable ionizing radiation at an effective rate or at a low rate for a time effective to sterilize said biological material.

**2.** A method for sterilizing a biological material that is sensitive to ionizing radiation, said method comprising:

(i) reducing the residual solvent content of a biological material to a level effective to protect said biological material from said ionizing radiation; and
(ii) irradiating said biological material with a suitable ionizing radiation at an effective rate or at a low rate for a time effective to sterilize said biological material.

**3.** A method for sterilizing a biological material that is sensitive to ionizing radiation, said method comprising:

(i) reducing the residual solvent content of a biological material to a level effective to protect said biological material from said ionizing radiation;
(ii) adding to said biological material at least one stabilizer in an amount effective to protect said biological material from said ionizing radiation; and
(iii) irradiating said biological material with a suitable ionizing radiation at an effective rate for a time effective to sterilize said biological material,

wherein steps (i) and (ii) may be performed in inverse order.

**4.** The method according to claim 1, further comprising the step of reducing the residual solvent content of said biological material prior to said step of irradiating said biological material.

**5.** The method according to claim 2, 3 or 4, wherein said solvent is water, or wherein said solvent is an organic solvent.

**6.** The method according to claim 5, wherein said residual water content is reduced by the addition of an organic solvent.

**7.** The method according to claims 1-6, wherein said biological material is suspended in an organic solvent following reduction of said residual solvent content.

**8.** The method according to claims 1-7, wherein said biological material is blood or a component of blood, a proteinaceous material, a proteinaceous material is a component of blood, a clotting factor, albumin, urokinase, polyclonal immunoglobulins, monoclonal immunoglobulins, or mixtures of one or more polyclonal and/or monoclonal immunoglobulins,
mammalian tissue or a component of or processed mammalian tissue, bone or a component of or processed bone, demineralized bone matrix, a recombinantly-produced biological material, a transgenic biological material, a food or a botanical product, a carbohydrate or polysaccharide, chitin, chitosan, NOCC-chitosan and derivatives thereof, or a product of cellular metabolism.

**9.** The method according to claim 8, wherein said clotting factor is selected from the group consisting of: Thrombin, Factor II, Factor V, Factor VII, Factor VIIa, Factor VIII, Factor IX, Factor X, Factor XIII, Factor XIIIa, Von Willebrand's Factor and Fibrinogen.

**10.** The method according to claim 8, wherein said immunoglobulins are immunoglobulin G, immunoglobulin M, or mixtures thereof.

**11.** The method according to claims 1-10, wherein said effective rate is not more than about 3.0 kGy/hour, or not more than about 2.0 kGy/hr, or not more than about 1.0 kGy/hr, or not more than about 0.3 kGy/hr.

**12.** The method according to claims 1-10, wherein said effective rate is more than about 3.0 kGy/hour, or at least about 6.0 kGy/hour, or at least about 18.0 kGy/hour, at least about 30.0 kGy/hour.

**13.** The method according to claims 1-12, wherein said biological material is maintained in a low oxygen atmosphere.

**14.** The method according to claim 13, wherein said biological material is maintained in an argon atmosphere.

**15.** The method according to claims 2-14, wherein said residual solvent content is reduced by lyophilization.

**16.** The method according to claims 2-15, wherein said residual solvent content is less than about 10.0%, or less than about 5.0%, or less than about 2.0%, or less than about 1.0%, or less than about 0.5%.

**17.** The method according to claims 1-16, wherein at least one sensitizer is added to said biological material prior to said step of irradiating said biological material.

**18.** The method according to claims 1-17, wherein said biological material contains at least one prion as a biological contaminant, or at least one virus as a biological contaminant.

**19.** The method according to claims 1 or 3-18, wherein said at least one stabilizer is an antioxidant.

**20.** The method according to claims 1 or 3-19, wherein said at least one stabilizer is a free radical scavenger.

**21.** The method according to claims 1 or 3-19, wherein said at least one stabilizer reduces damage due to reactive oxygen species.

**22.** The method according to claims 1 or 3-19, wherein said at least one stabilizer is selected from the group consisting of: ascorbic acid or a salt or ester thereof; glutathione; 6-hydroxy-2, 5, 7, 8-tetramethylchroman-2-carboxylic acid; uric acid or a salt or ester thereof; methionine; histidine; N-acetyl cysteine; and mixtures of two or more of said stabilizers.

**23.** The method according to claim 22, wherein said mixtures of two or more of said stabilizers is selected from the group consisting of: mixtures of ascorbic acid, or a salt or ester thereof, and uric acid, or a salt or ester thereof; mixtures of ascorbic acid, or a salt or ester thereof, and 6-hydroxy-2, 5, 7, 8-tetramethylchroman-2-carboxylic acid; mixtures of ascorbic acid, or a salt or ester thereof, uric acid, or a salt or ester thereof, and 6-hydroxy-2, 5, 7, 8 tetramethylchroman-2-carboxylic acid; and mixtures of uric acid, or a salt or ester thereof and 6-hydroxy-2, 5, 7, 8-tetramethylchroman-2-carboxylic acid.

**24.** The method according to claims 1-23, wherein said ionizing radiation is corpuscular radiation or electromagnetic radiation or a mixture thereof, or wherein said ionizing radiation is e-beam radiation.

**25.** The method according to claim 24, wherein said electromagnetic radiation is selected from the group consisting of radio waves, visible and invisible light, ultraviolet light, x-ray radiation, and gamma radiation.

**26.** The method according to claims 1-23, wherein said ionizing radiation is gamma radiation.

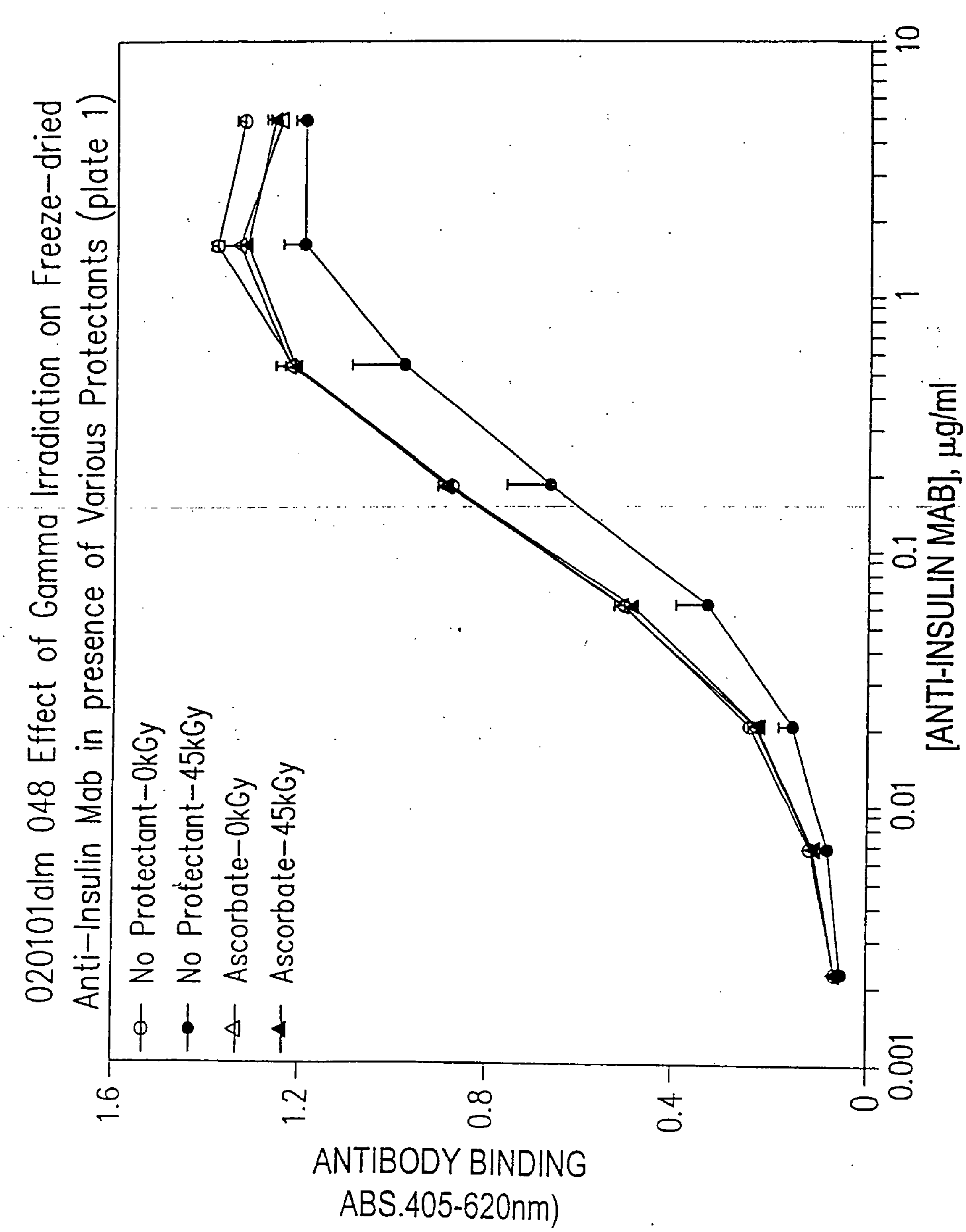
020101alm 048 Effect of Gamma Irradiation on Freeze-dried
Anti-Insulin Mab in presence of Various Protectants (plate 1)
No Protectant-0kGy
No Protectant-45kGy
Ascorbate-0kGy
Ascorbate-45kGy
1.6
1.2
0.8
0.4
0
0.001
0.01
0.1
1
10
[ANTI-INSULIN MAB], µg/ml
ANTIBODY BINDING
ABS.405-620nm)

Fig. 1

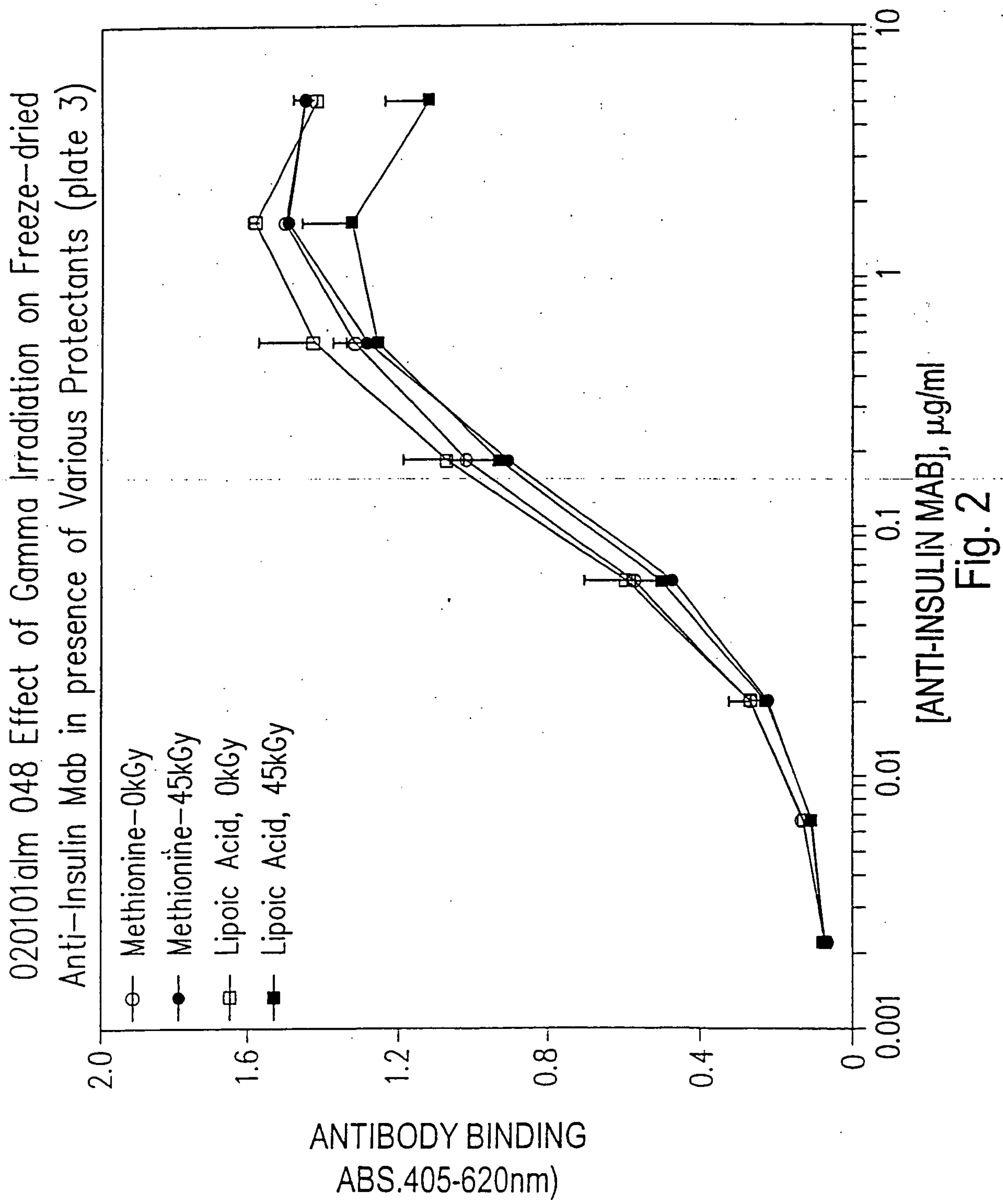
020101alm 048 Effect of Gamma Irradiation on Freeze-dried
Anti-Insulin Mab in presence of Various Protectants (plate 3)
Methionine-0kGy
Methionine-45kGy
Lipoic Acid, 0kGy
Lipoic Acid, 45kGy
2.0
1.6
1.2
0.8
0.4
0
0.001
0.01
0.1
1
10
ANTIBODY BINDING
ABS.405-620nm)
[ANTI-INSULIN MAB], µg/ml

Fig. 2

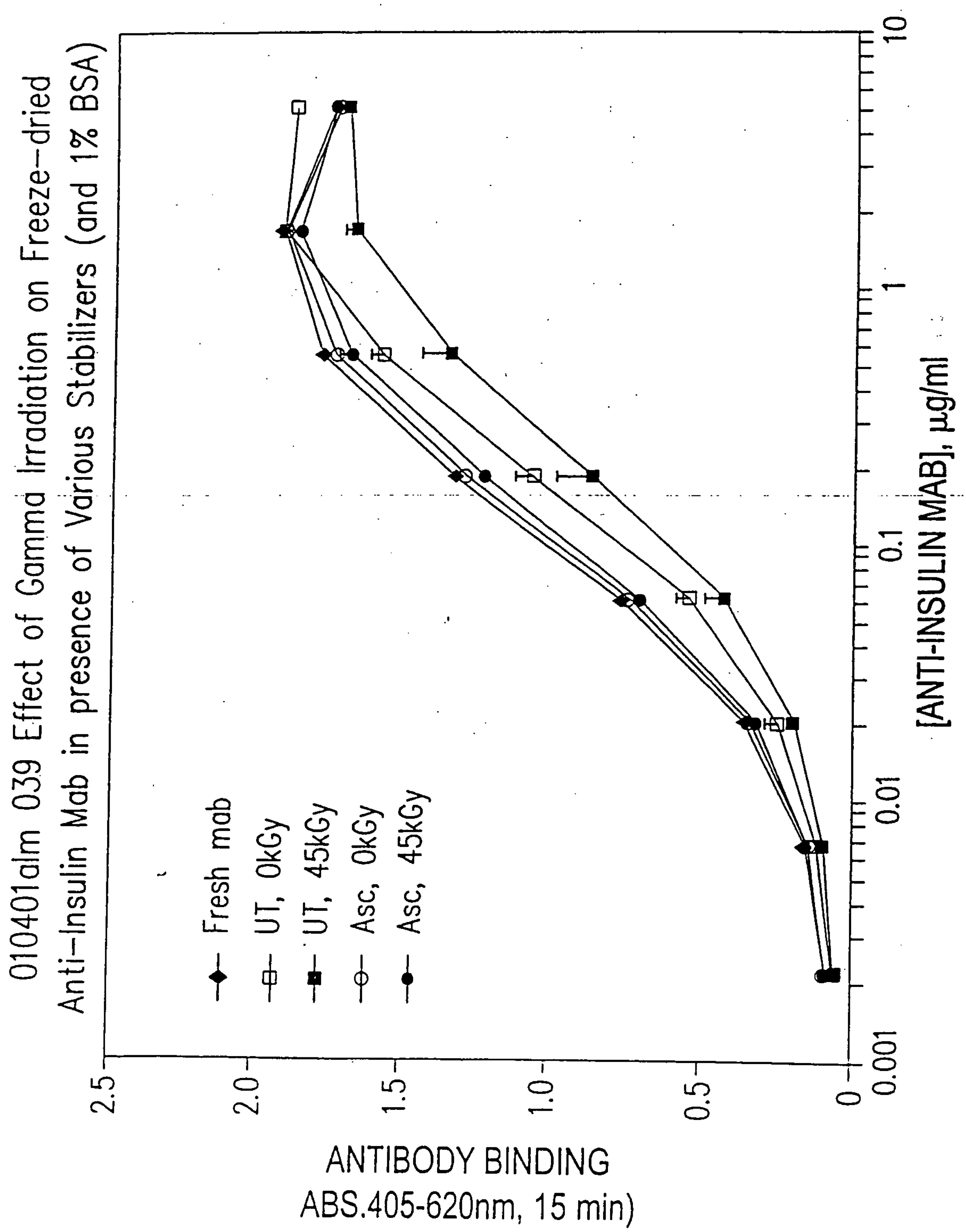
010401alm 039 Effect of Gamma Irradiation on Freeze-dried Anti-Insulin Mab in presence of Various Stabilizers (and 1% BSA)
Fresh mab
UT, 0kGy
UT, 45kGy
Asc, 0kGy
Asc, 45kGy
ANTIBODY BINDING
ABS.405-620nm, 15 min)
2.5
2.0
1.5
1.0
0.5
0
0.001
0.01
0.1
1
10
[ANTI-INSULIN MAB], µg/ml

Fig. 3A

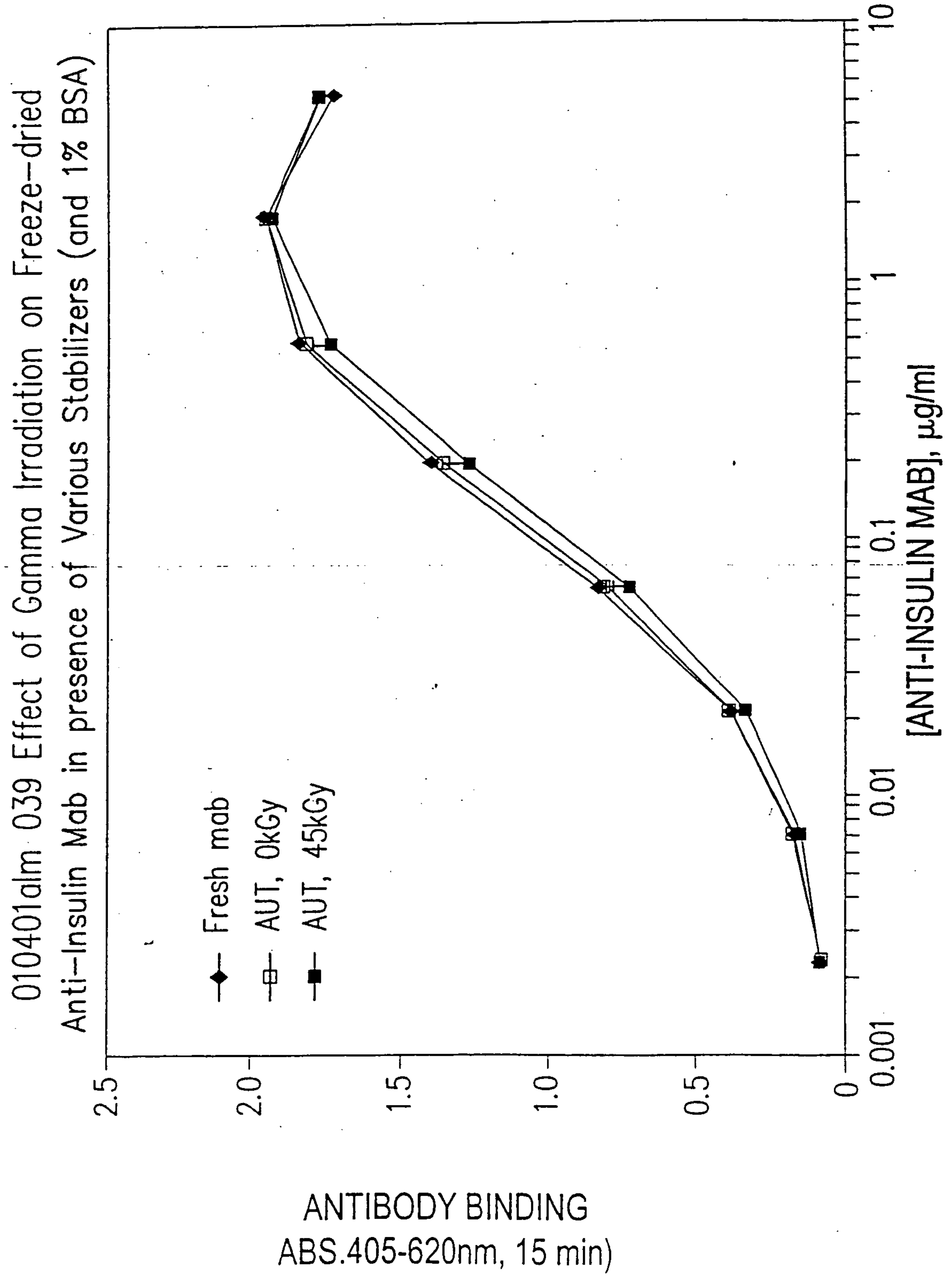
010401alm 039 Effect of Gamma Irradiation on Freeze-dried
Anti-Insulin Mab in presence of Various Stabilizers (and 1% BSA)
Fresh mab
AUT, 0kGy
AUT, 45kGy
2.5
2.0
1.5
1.0
0.5
0
0.001
0.01
0.1
1
10
[ANTI-INSULIN MAB], µg/ml
ANTIBODY BINDING
ABS.405-620nm, 15 min)

Fig. 3B

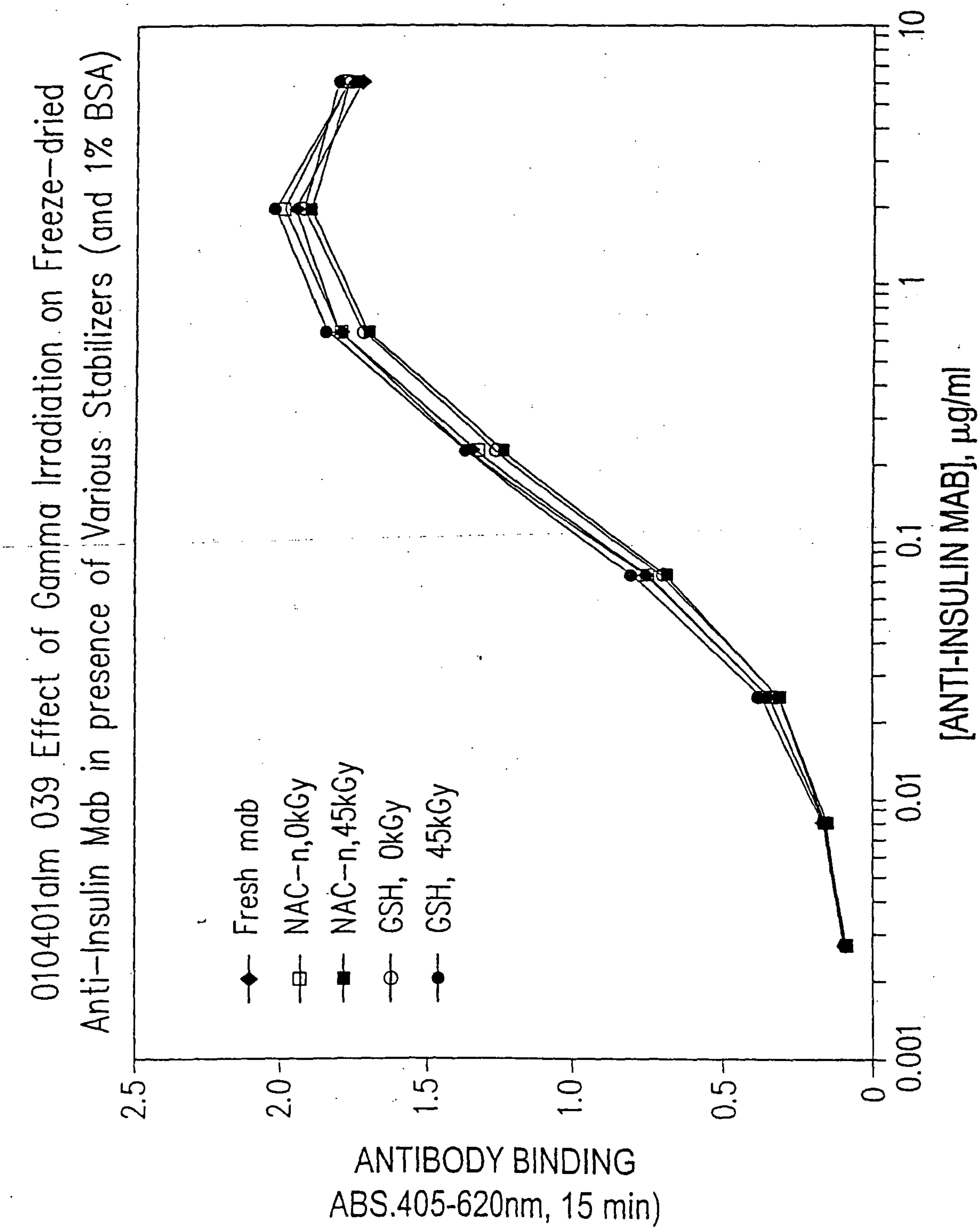
010401alm 039 Effect of Gamma Irradiation on Freeze-dried
Anti-Insulin Mab in presence of Various Stabilizers (and 1% BSA)
Fresh mab
NAC-n,0kGy
NAC-n,45kGy
GSH, 0kGy
GSH, 45kGy
2.5
2.0
1.5
1.0
0.5
0
0.001
0.01
0.1
1
10
ANTIBODY BINDING
ABS.405-620nm, 15 min)
[ANTI-INSULIN MAB], µg/ml

Fig. 3C

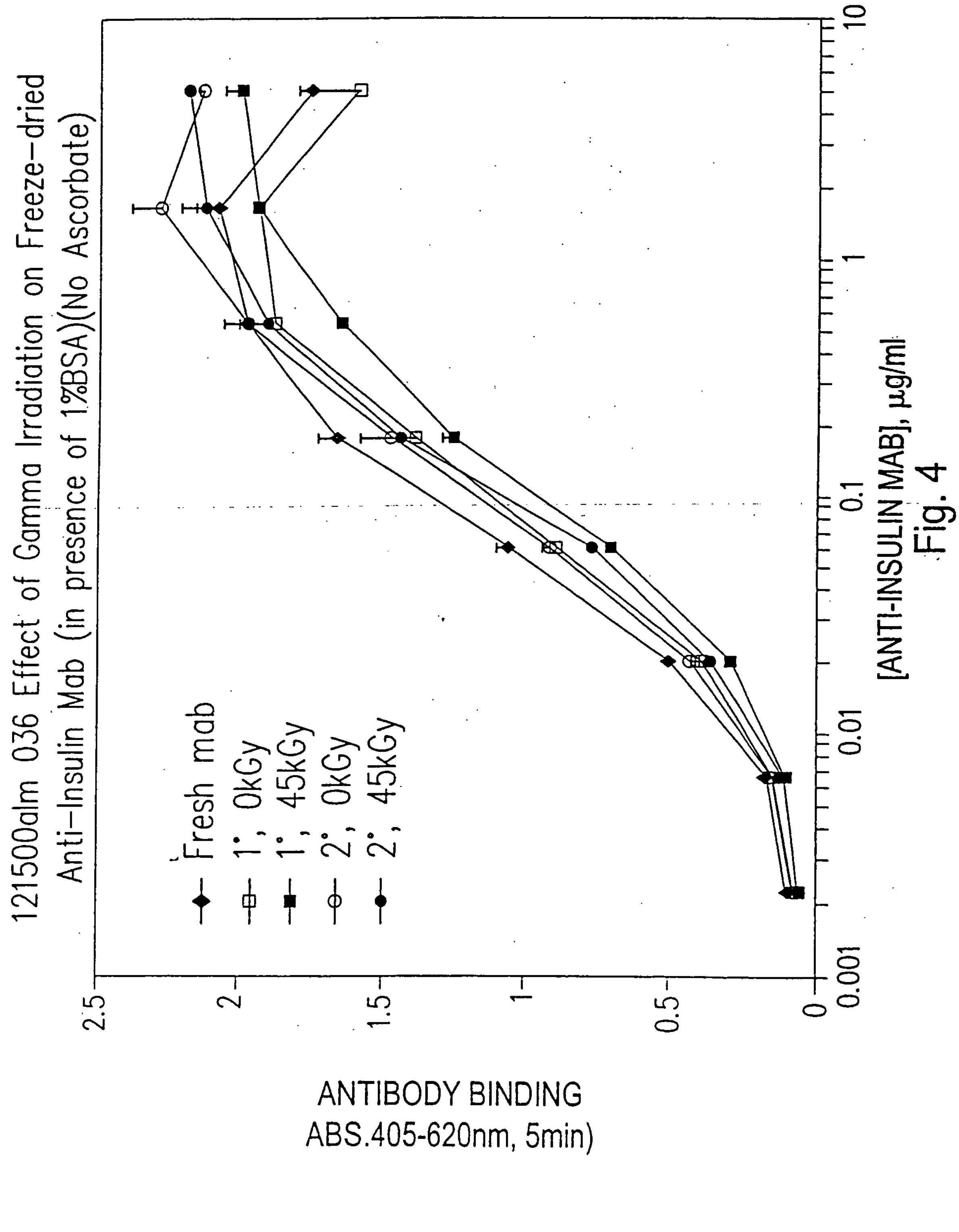

121500alm 036 Effect of Gamma Irradiation on Freeze-dried
Anti-Insulin Mab (in presence of 1%BSA)(No Ascorbate)
Fresh mab
1°, 0kGy
1°, 45kGy
2°, 0kGy
2°, 45kGy
2.5
2
1.5
1
0.5
0
0.001
0.01
0.1
1
10
[ANTI-INSULIN MAB], µg/ml
ANTIBODY BINDING
ABS.405-620nm, 5min)


Fig. 4

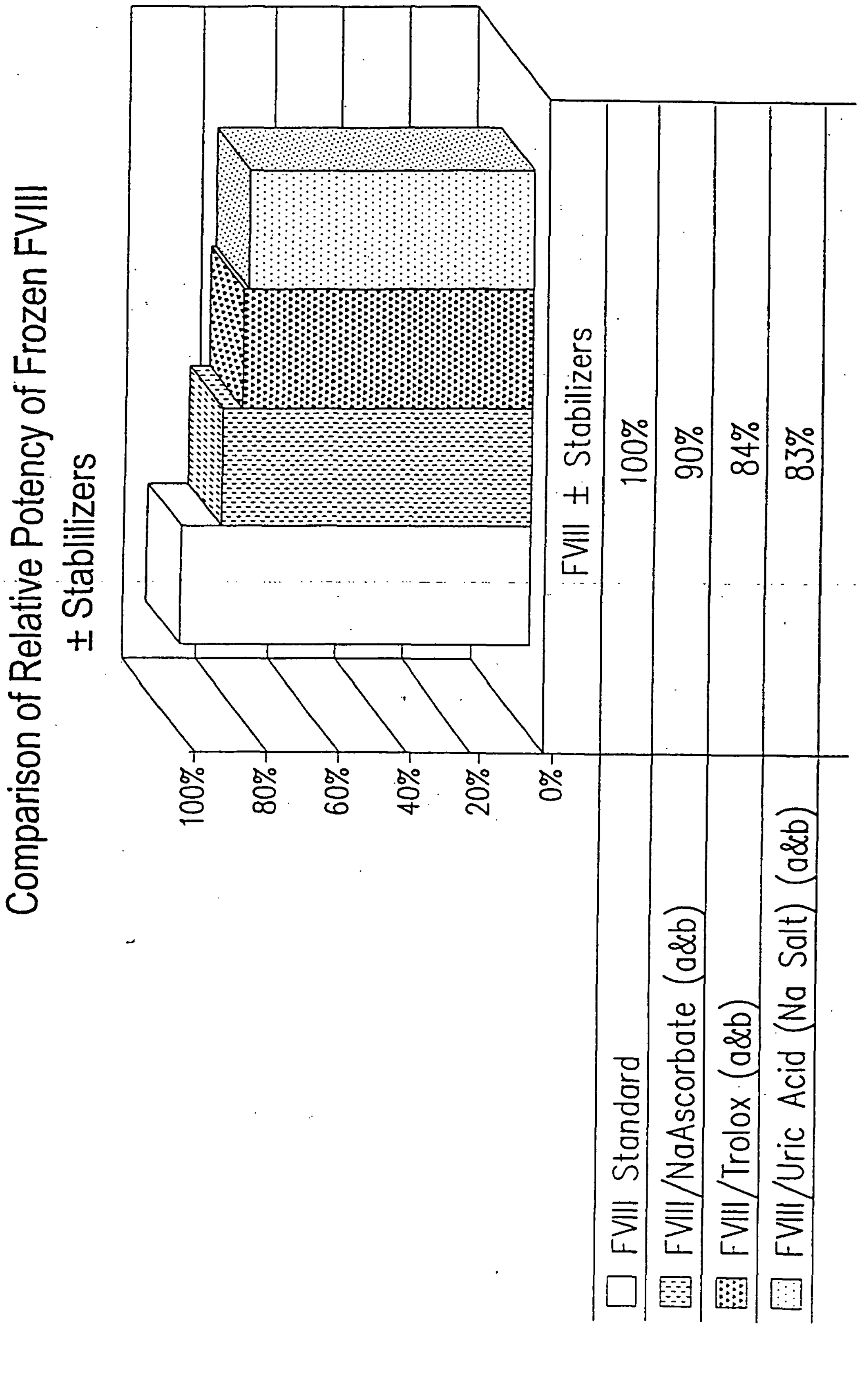
Comparison of Relative Potency of Frozen FVIII
± Stabilizers
100%
80%
60%
40%
20%
0%
FVIII ± Stabilizers
100%
90%
84%
83%
FVIII Standard
FVIII/NaAscorbate (a&b)
FVIII/Trolox (a&b)
FVIII/Uric Acid (Na Salt) (a&b)

Fig. 5

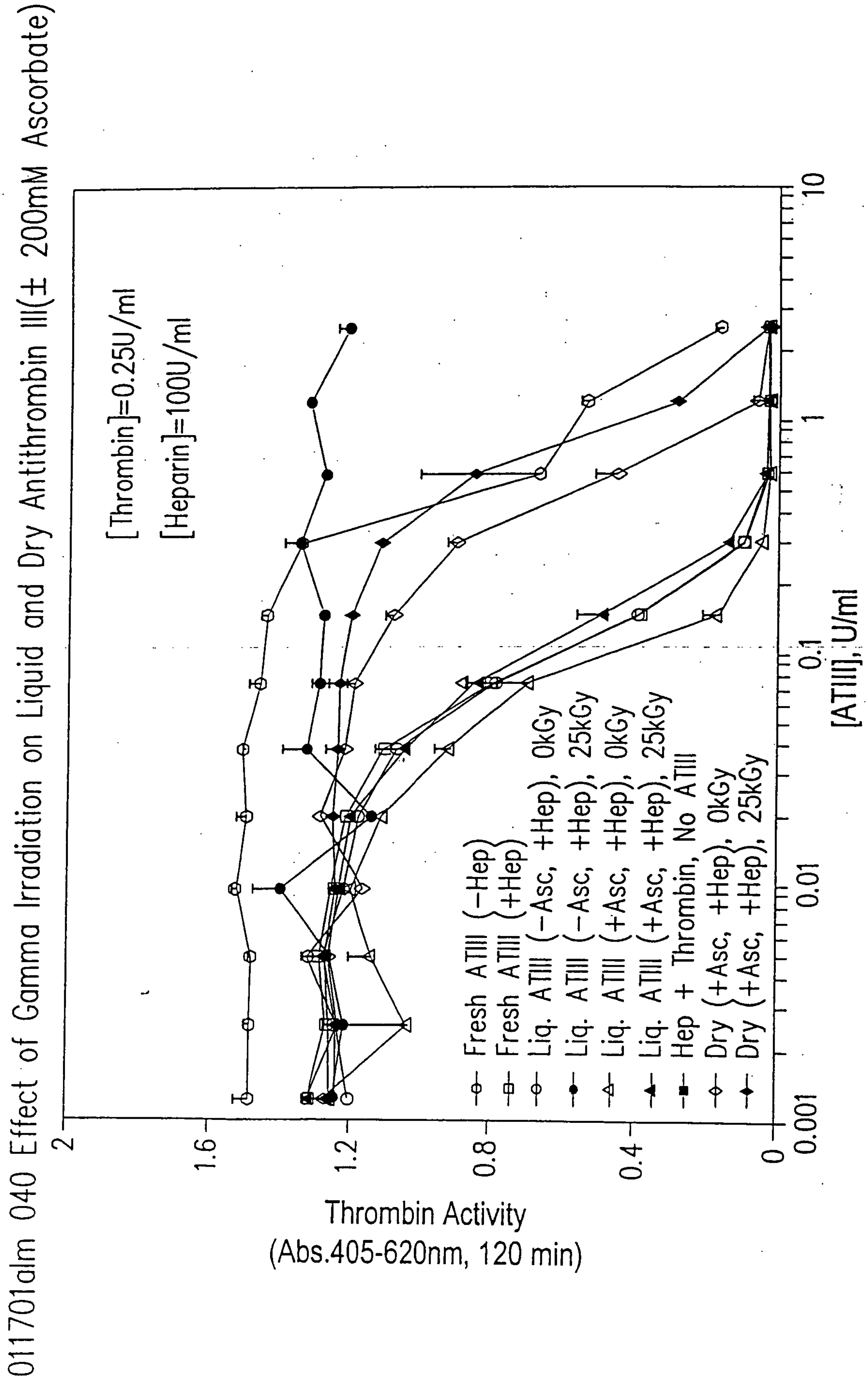
011701alm 040 Effect of Gamma Irradiation on Liquid and Dry Antithrombin III(± 200mM Ascorbate)
[Thrombin]=0.25U/ml
[Heparin]=100U/ml
Fresh ATIII (−Hep)
Fresh ATIII (+Hep)
Liq. ATIII (−Asc, +Hep), 0kGy
Liq. ATIII (−Asc, +Hep), 25kGy
Liq. ATIII (+Asc, +Hep), 0kGy
Liq. ATIII (+Asc, +Hep), 25kGy
Hep + Thrombin, No ATIII
Dry (+Asc, +Hep), 0kGy
Dry (+Asc, +Hep), 25kGy
Thrombin Activity
(Abs.405-620nm, 120 min)
2
1.6
1.2
0.8
0.4
0
0.001
0.01
0.1
1
10
[ATIII], U/ml

Fig. 6

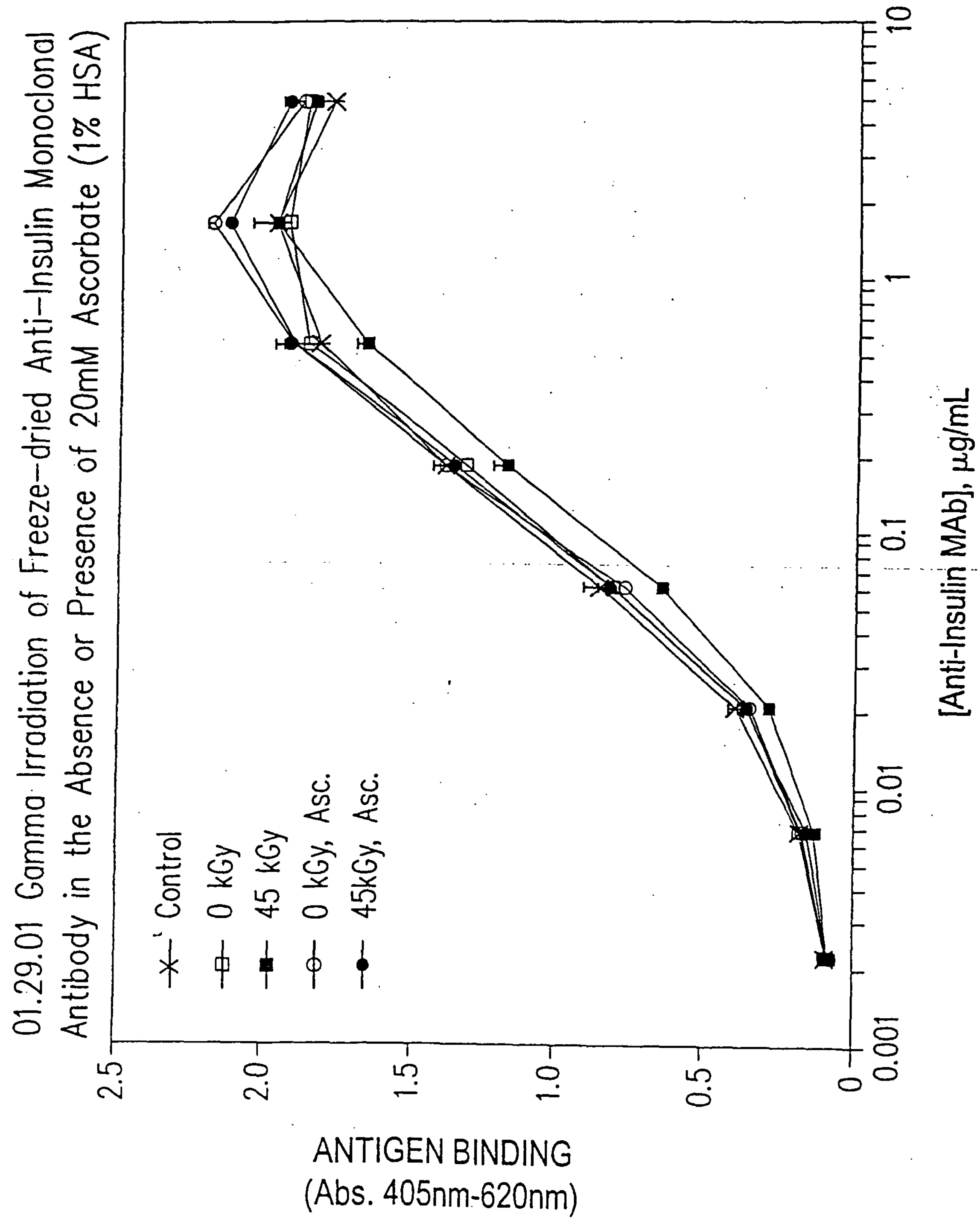

01.29.01 Gamma Irradiation of Freeze-dried Anti-Insulin Monoclonal Antibody in the Absence or Presence of 20mM Ascorbate (1% HSA)
Control
0 kGy
45 kGy
0 kGy, Asc.
45kGy, Asc.
ANTIGEN BINDING (Abs. 405nm-620nm)
2.5
2.0
1.5
1.0
0.5
0
0.001
0.01
0.1
1
10
[Anti-Insulin MAb], μg/mL


Fig. 7

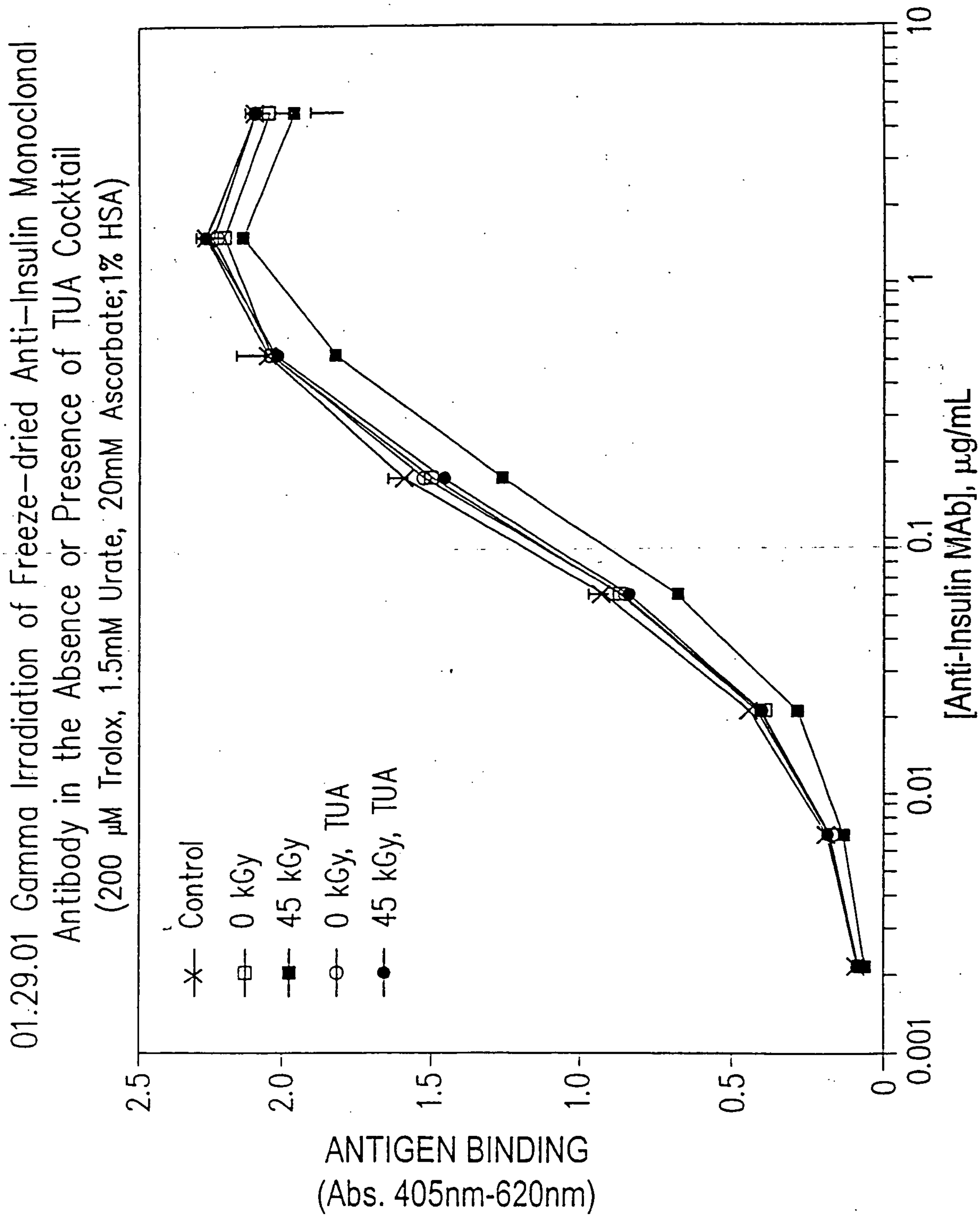
01.29.01 Gamma Irradiation of Freeze-dried Anti-Insulin Monoclonal
Antibody in the Absence or Presence of TUA Cocktail
(200 μM Trolox, 1.5mM Urate, 20mM Ascorbate;1% HSA)
Control
0 kGy
45 kGy
0 kGy, TUA
45 kGy, TUA
ANTIGEN BINDING
(Abs. 405nm-620nm)
2.5
2.0
1.5
1.0
0.5
0
0.001
0.01
0.1
1
10
[Anti-Insulin MAb], μg/mL

Fig. 8

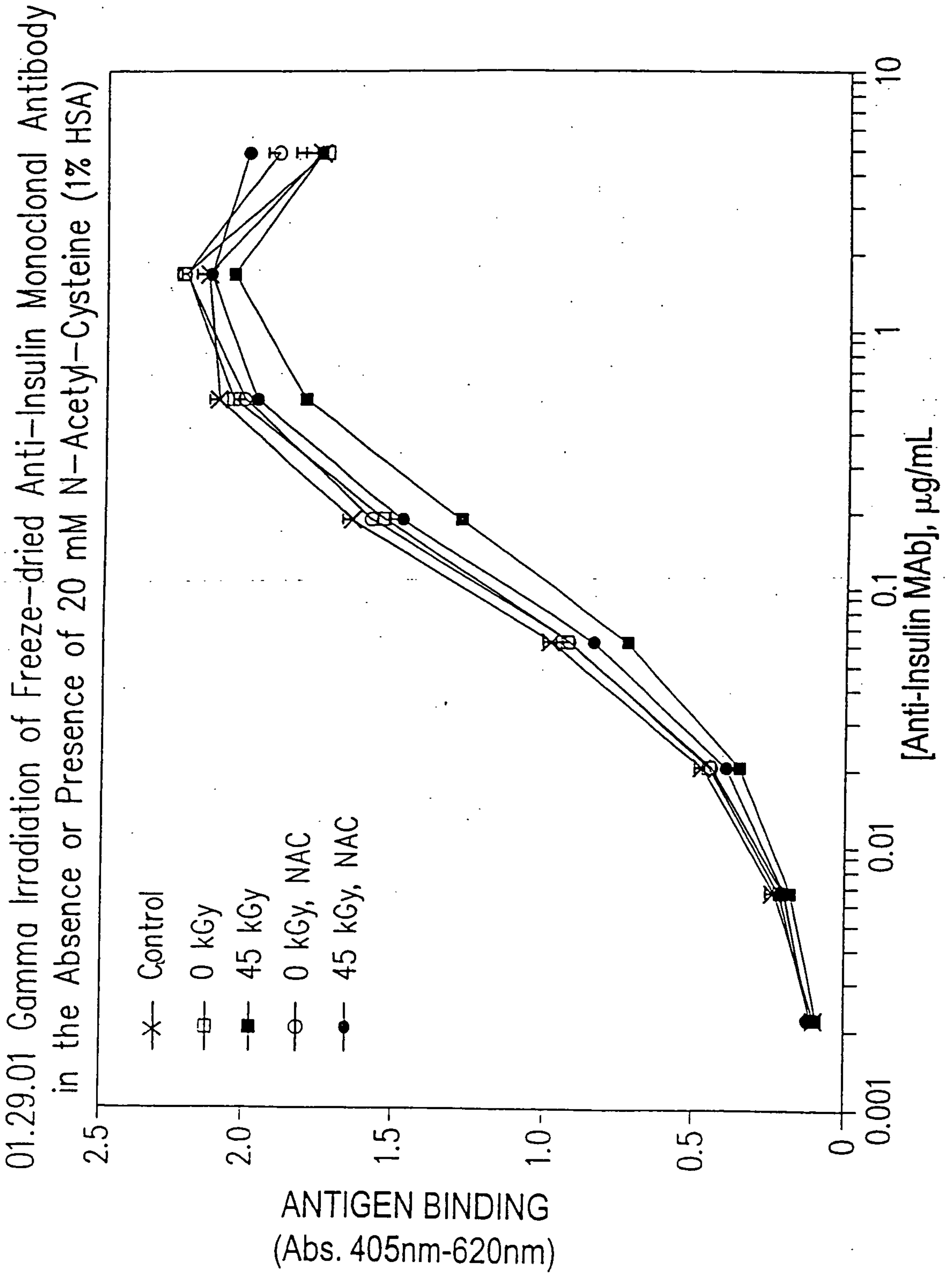
01.29.01 Gamma Irradiation of Freeze-dried Anti-Insulin Monoclonal Antibody
in the Absence or Presence of 20 mM N-Acetyl-Cysteine (1% HSA)
Control
0 kGy
45 kGy
0 kGy, NAC
45 kGy, NAC
ANTIGEN BINDING
(Abs. 405nm-620nm)
0
0.5
1.0
1.5
2.0
2.5
0.001
0.01
0.1
1
10
[Anti-Insulin MAb], µg/mL

Fig. 9

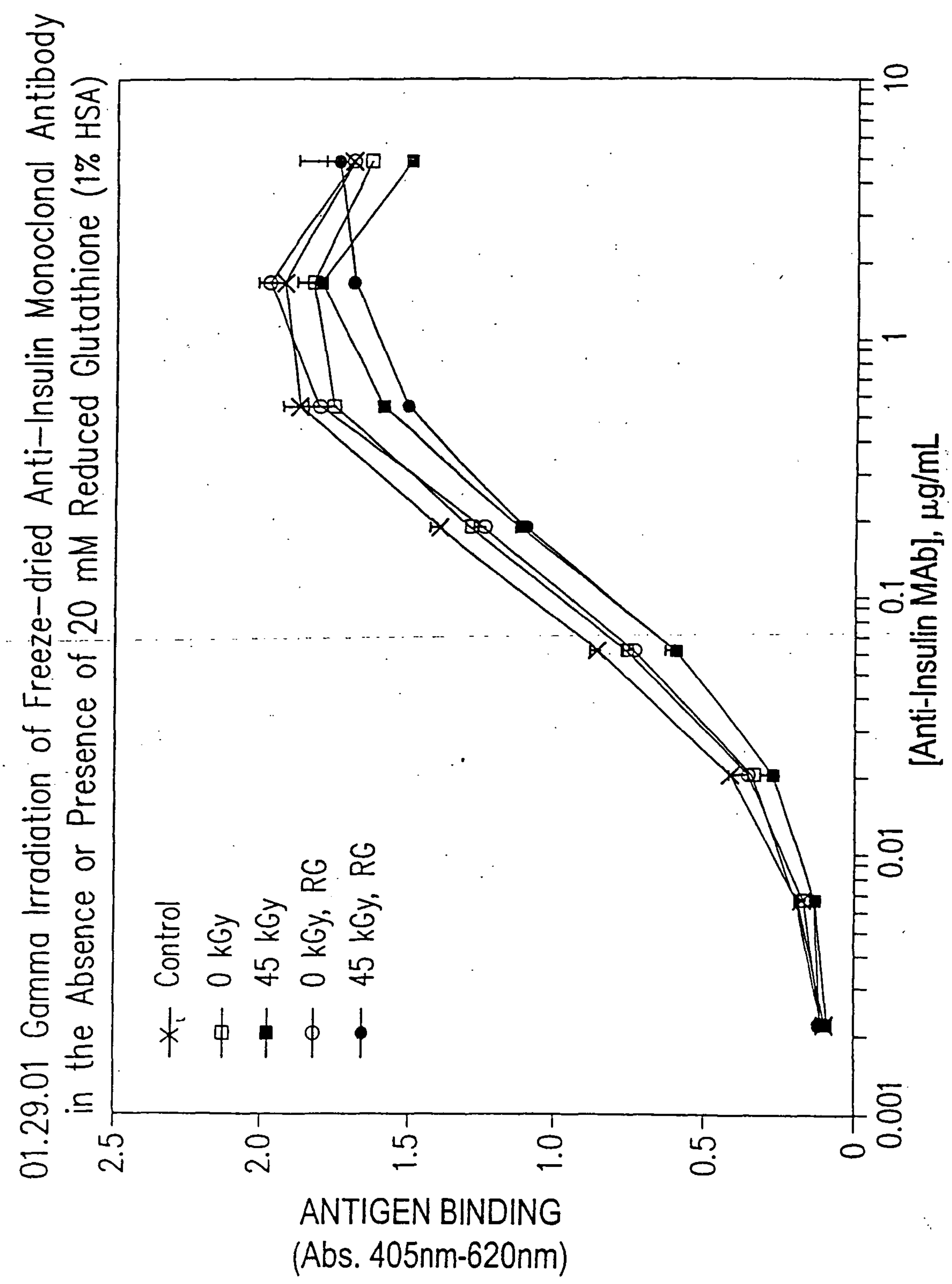
01.29.01 Gamma Irradiation of Freeze-dried Anti-Insulin Monoclonal Antibody
in the Absence or Presence of 20 mM Reduced Glutathione (1% HSA)
Control
0 kGy
45 kGy
0 kGy, RG
45 kGy, RG
ANTIGEN BINDING
(Abs. 405nm-620nm)
2.5
2.0
1.5
1.0
0.5
0
0.001
0.01
0.1
1
10
[Anti-Insulin MAb], µg/mL

Fig. 10

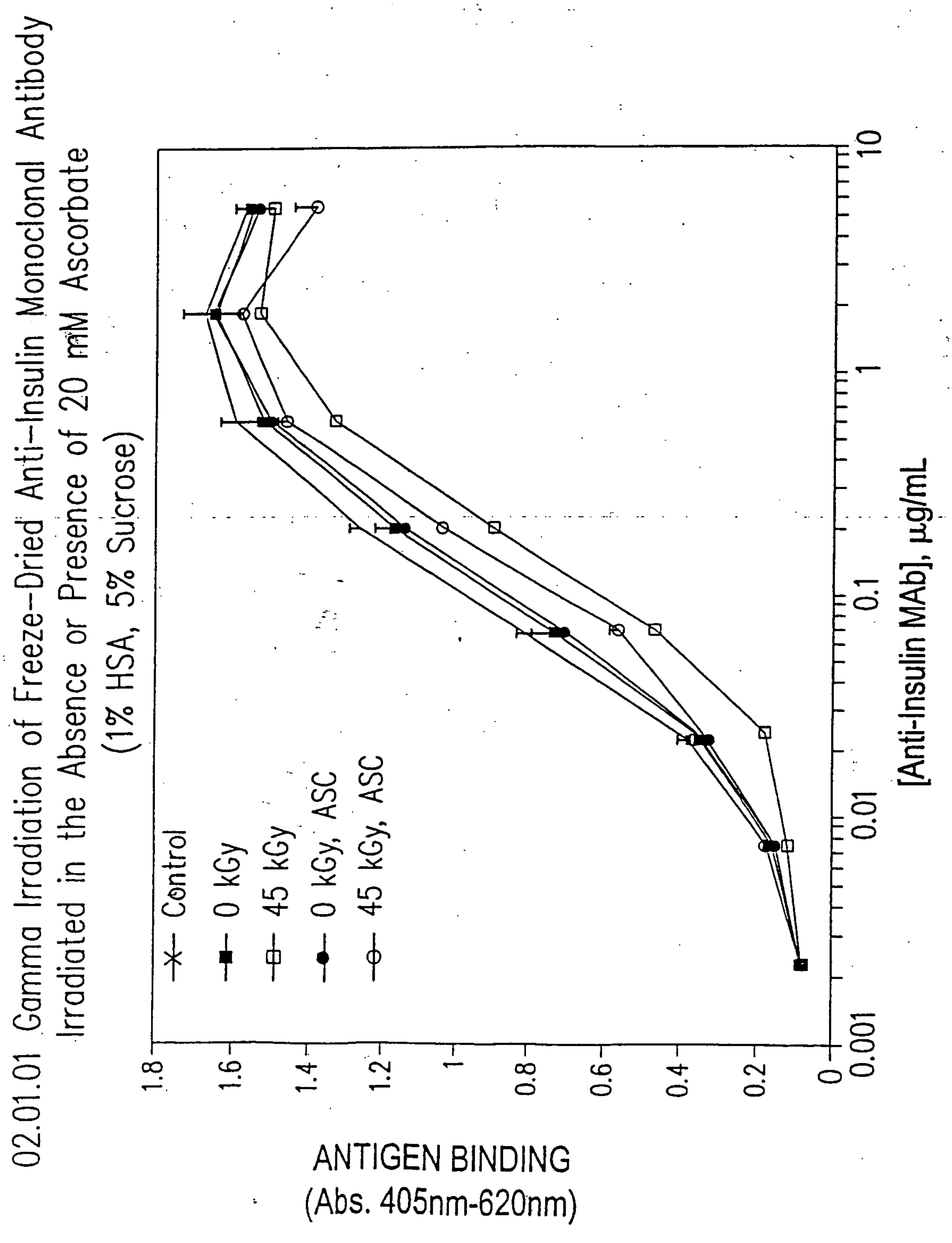
02.01.01 Gamma Irradiation of Freeze-Dried Anti-Insulin Monoclonal Antibody
Irradiated in the Absence or Presence of 20 mM Ascorbate
(1% HSA, 5% Sucrose)
Control
0 kGy
45 kGy
0 kGy, ASC
45 kGy, ASC
ANTIGEN BINDING
(Abs. 405nm-620nm)
1.8
1.6
1.4
1.2
1
0.8
0.6
0.4
0.2
0
0.001
0.01
0.1
1
10
[Anti-Insulin MAb], µg/mL

Fig. 11

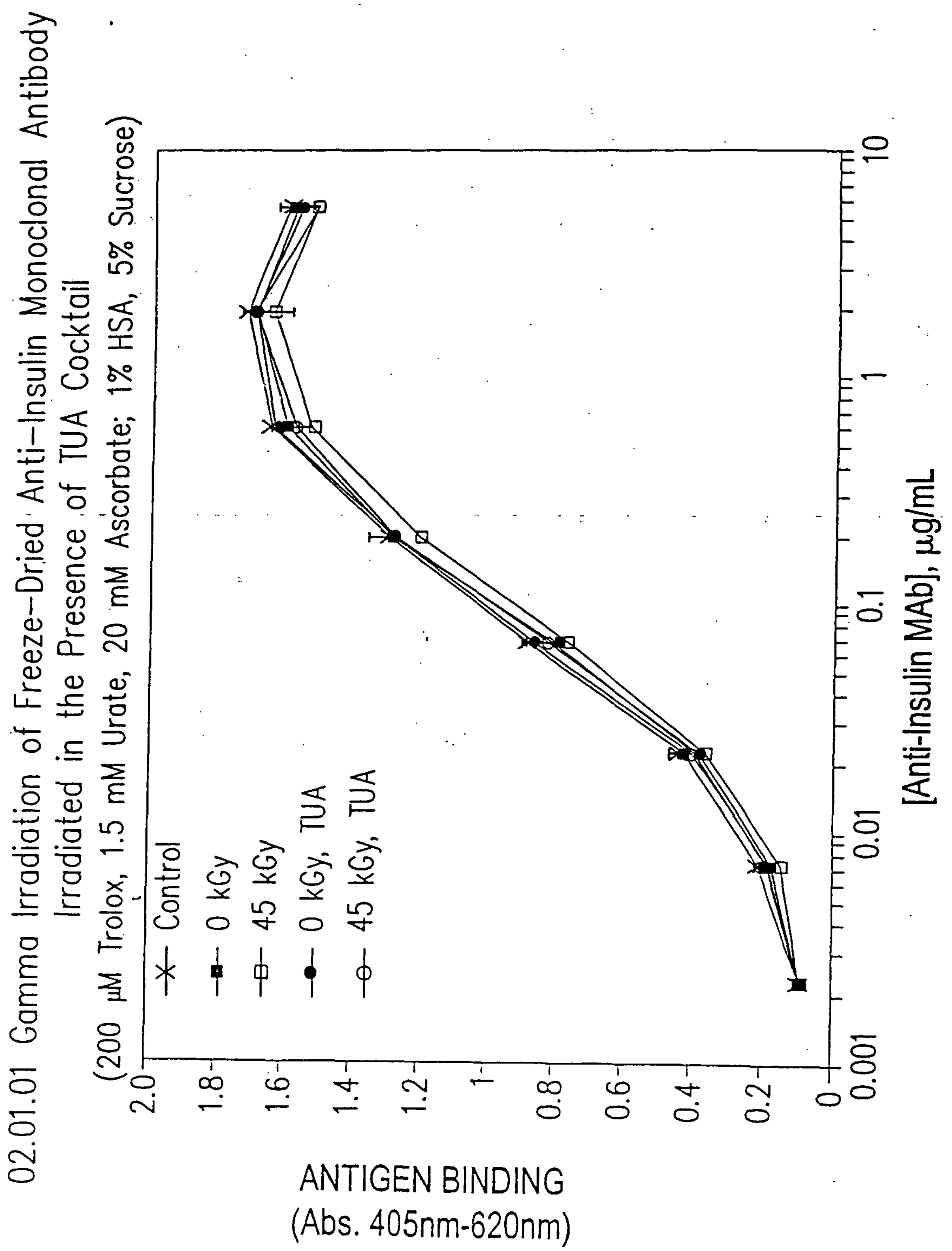

02.01.01 Gamma Irradiation of Freeze-Dried Anti-Insulin Monoclonal Antibody
Irradiated in the Presence of TUA Cocktail
(200 µM Trolox, 1.5 mM Urate, 20 mM Ascorbate; 1% HSA, 5% Sucrose)
Control
0 kGy
45 kGy
0 kGy, TUA
45 kGy, TUA
ANTIGEN BINDING
(Abs. 405nm-620nm)
2.0
1.8
1.6
1.4
1.2
1
0.8
0.6
0.4
0.2
0
0.001
0.01
0.1
1
10
[Anti-Insulin MAb], µg/mL


Fig. 12

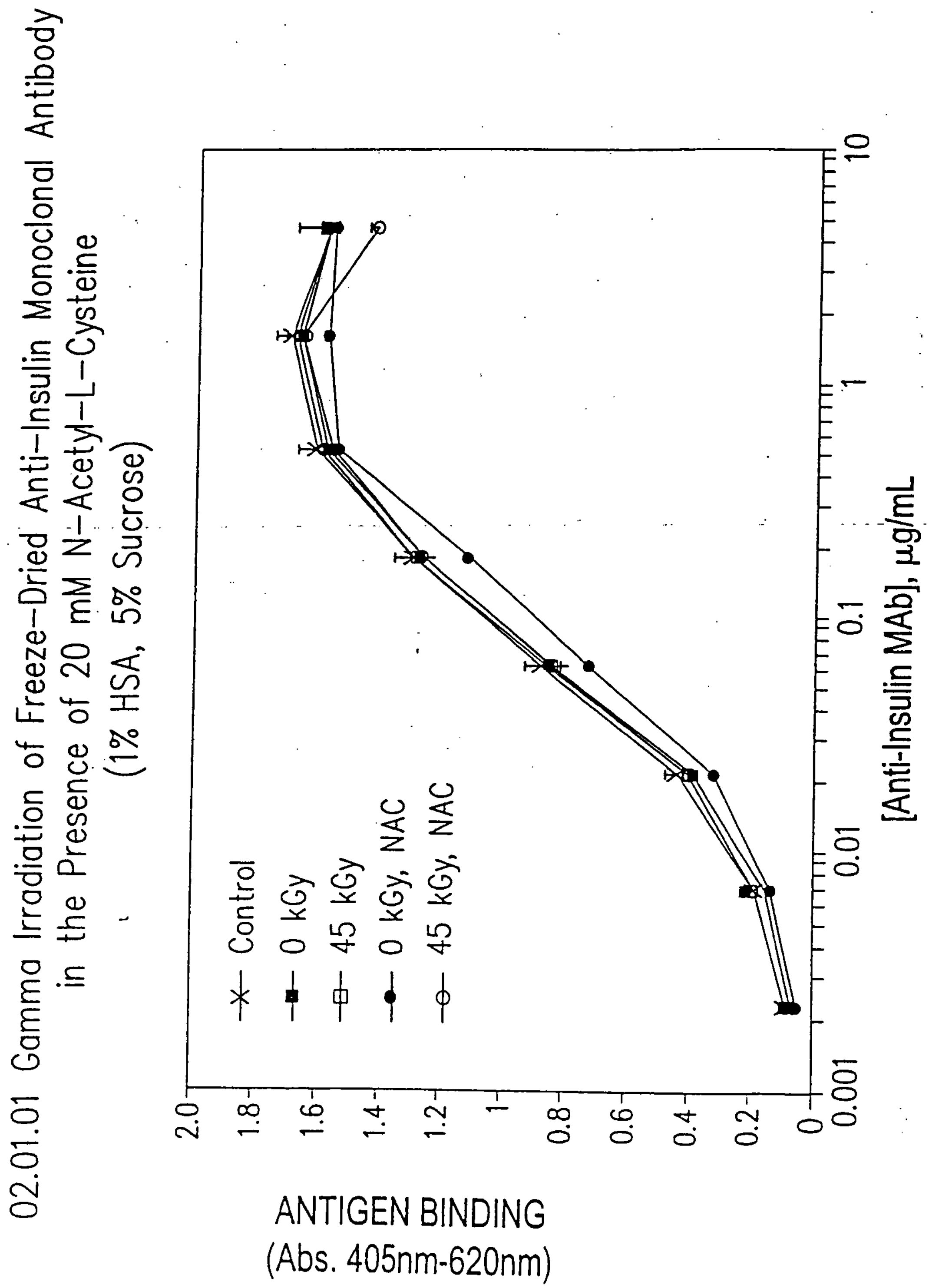
02.01.01 Gamma Irradiation of Freeze-Dried Anti-Insulin Monoclonal Antibody
in the Presence of 20 mM N-Acetyl-L-Cysteine
(1% HSA, 5% Sucrose)
ANTIGEN BINDING
(Abs. 405nm-620nm)
2.0
1.8
1.6
1.4
1.2
1
0.8
0.6
0.4
0.2
0
0.001
0.01
0.1
1
10
[Anti-Insulin MAb], µg/mL
Control
0 kGy
45 kGy
0 kGy, NAC
45 kGy, NAC

Fig. 13

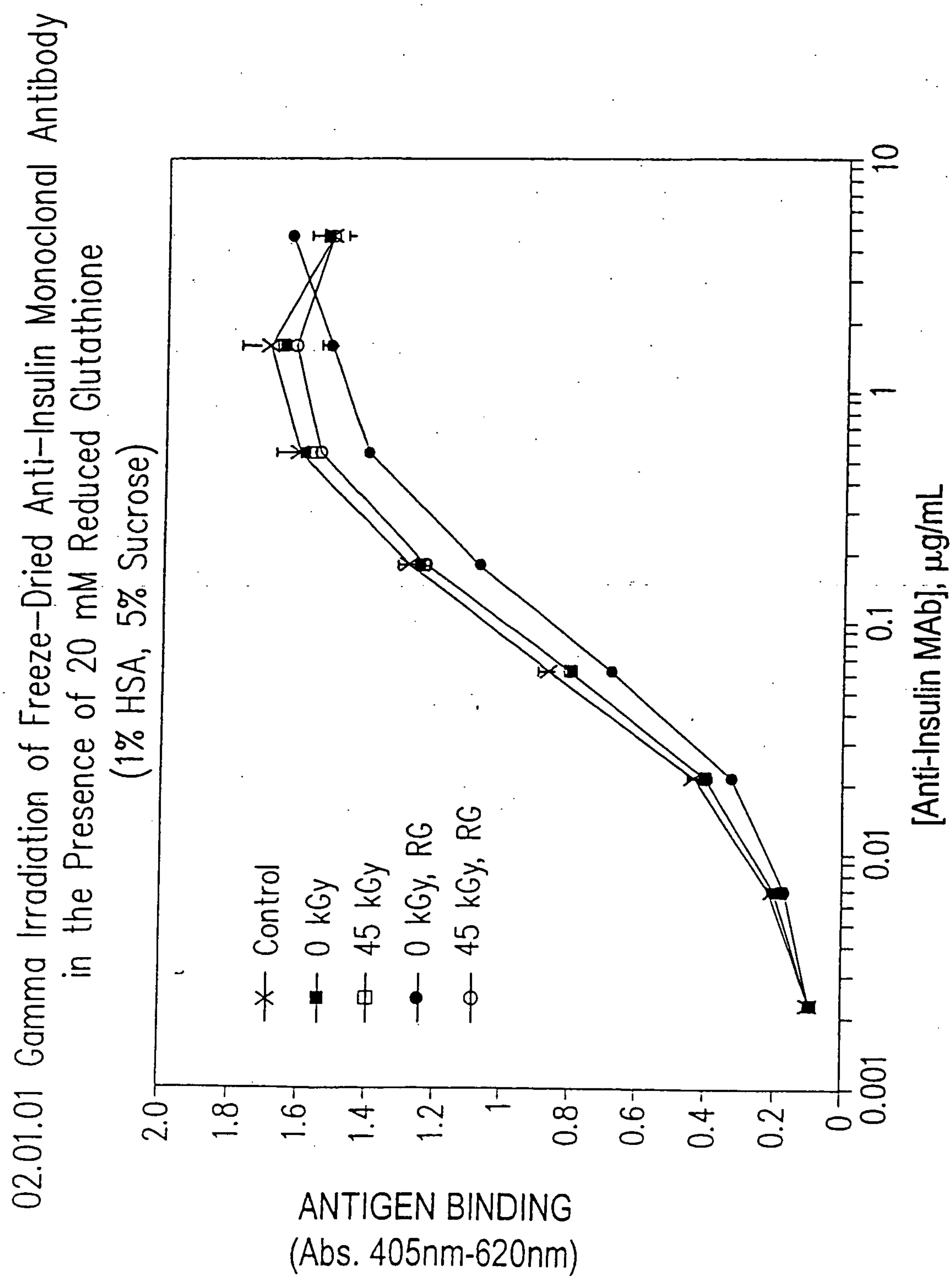
02.01.01 Gamma Irradiation of Freeze-Dried Anti-Insulin Monoclonal Antibody
in the Presence of 20 mM Reduced Glutathione
(1% HSA, 5% Sucrose)
ANTIGEN BINDING
(Abs. 405nm-620nm)
2.0
1.8
1.6
1.4
1.2
1
0.8
0.6
0.4
0.2
0
0.001
0.01
0.1
1
10
[Anti-Insulin MAb], µg/mL
Control
0 kGy
45 kGy
0 kGy, RG
45 kGy, RG

Fig. 14

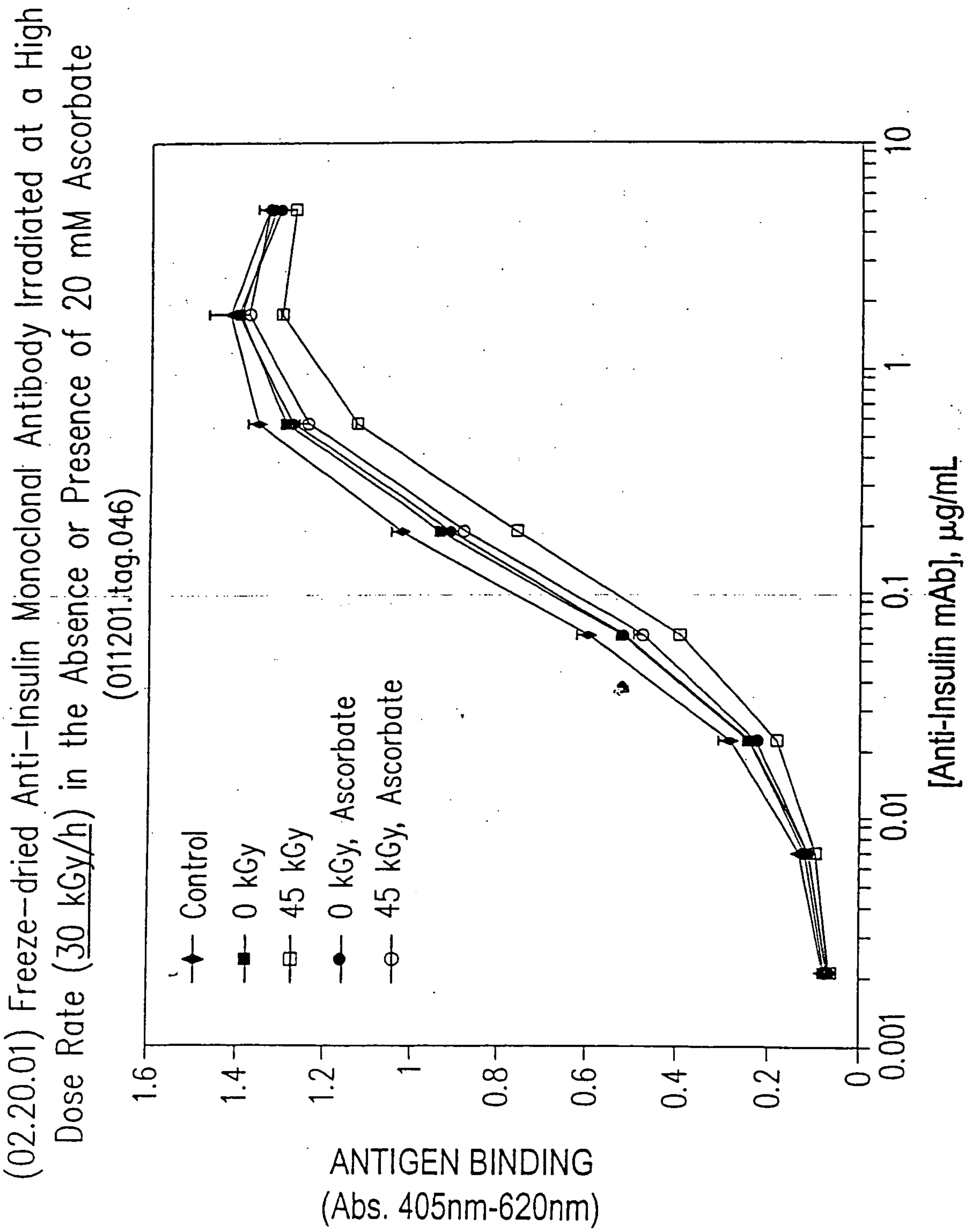
(02.20.01) Freeze-dried Anti-Insulin Monoclonal Antibody Irradiated at a High Dose Rate (30 kGy/h) in the Absence or Presence of 20 mM Ascorbate
(011201.tag.046)
Control
0 kGy
45 kGy
0 kGy, Ascorbate
45 kGy, Ascorbate
ANTIGEN BINDING
(Abs. 405nm-620nm)
0
0.2
0.4
0.6
0.8
1
1.2
1.4
1.6
0.001
0.01
0.1
1
10
[Anti-Insulin mAb], µg/mL

Fig. 15

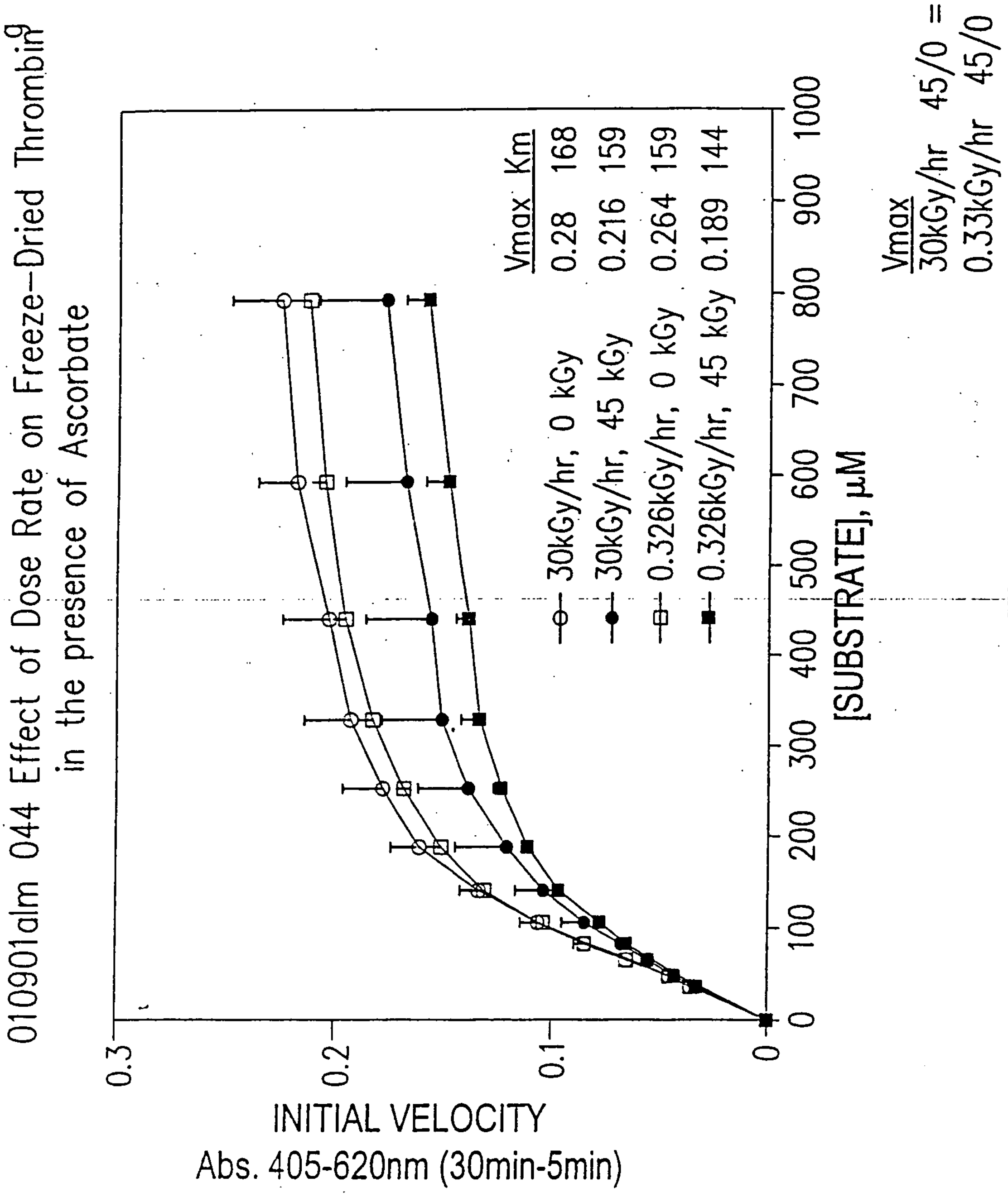
010901alm 044 Effect of Dose Rate on Freeze-Dried Thrombin:g
in the presence of Ascorbate
INITIAL VELOCITY
Abs. 405-620nm (30min-5min)
0.3
0.2
0.1
0
0
100
200
300
400
500
600
700
800
900
1000
[SUBSTRATE], μM
30kGy/hr, 0 kGy
30kGy/hr, 45 kGy
0.326kGy/hr, 0 kGy
0.326kGy/hr, 45 kGy
Vmax Km
0.28 168
0.216 159
0.264 159
0.189 144
Vmax
30kGy/hr 45/0 = 77% recovery
0.33kGy/hr 45/0 = 72% recovery

Fig. 16

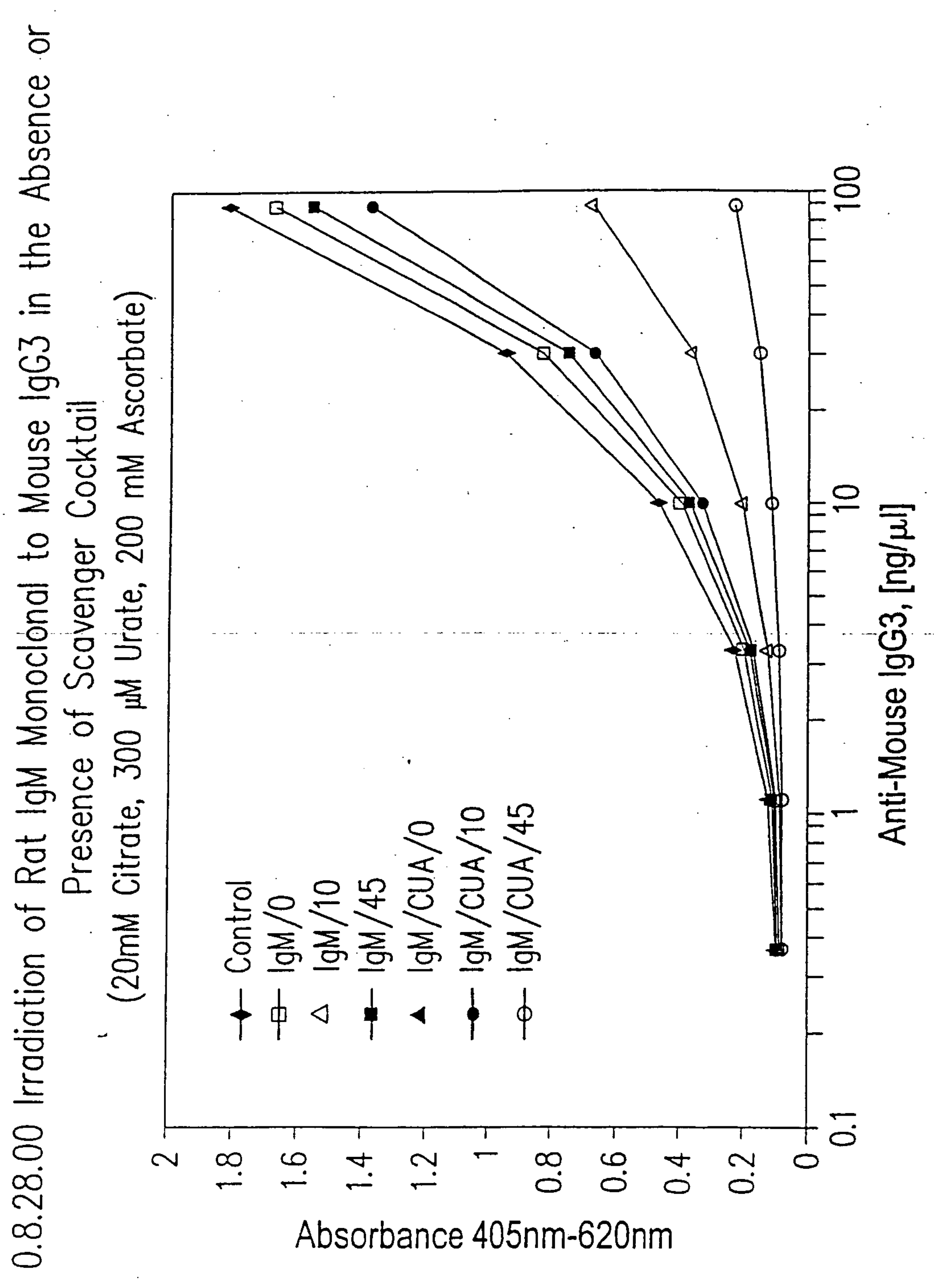
0.8.28.00 Irradiation of Rat IgM Monoclonal to Mouse IgG3 in the Absence or Presence of Scavenger Cocktail
(20mM Citrate, 300 µM Urate, 200 mM Ascorbate)
Control
IgM/0
IgM/10
IgM/45
IgM/CUA/0
IgM/CUA/10
IgM/CUA/45
Absorbance 405nm-620nm
2
1.8
1.6
1.4
1.2
1
0.8
0.6
0.4
0.2
0
0.1
1
10
100
Anti-Mouse IgG3, [ng/µl]

Fig. 17

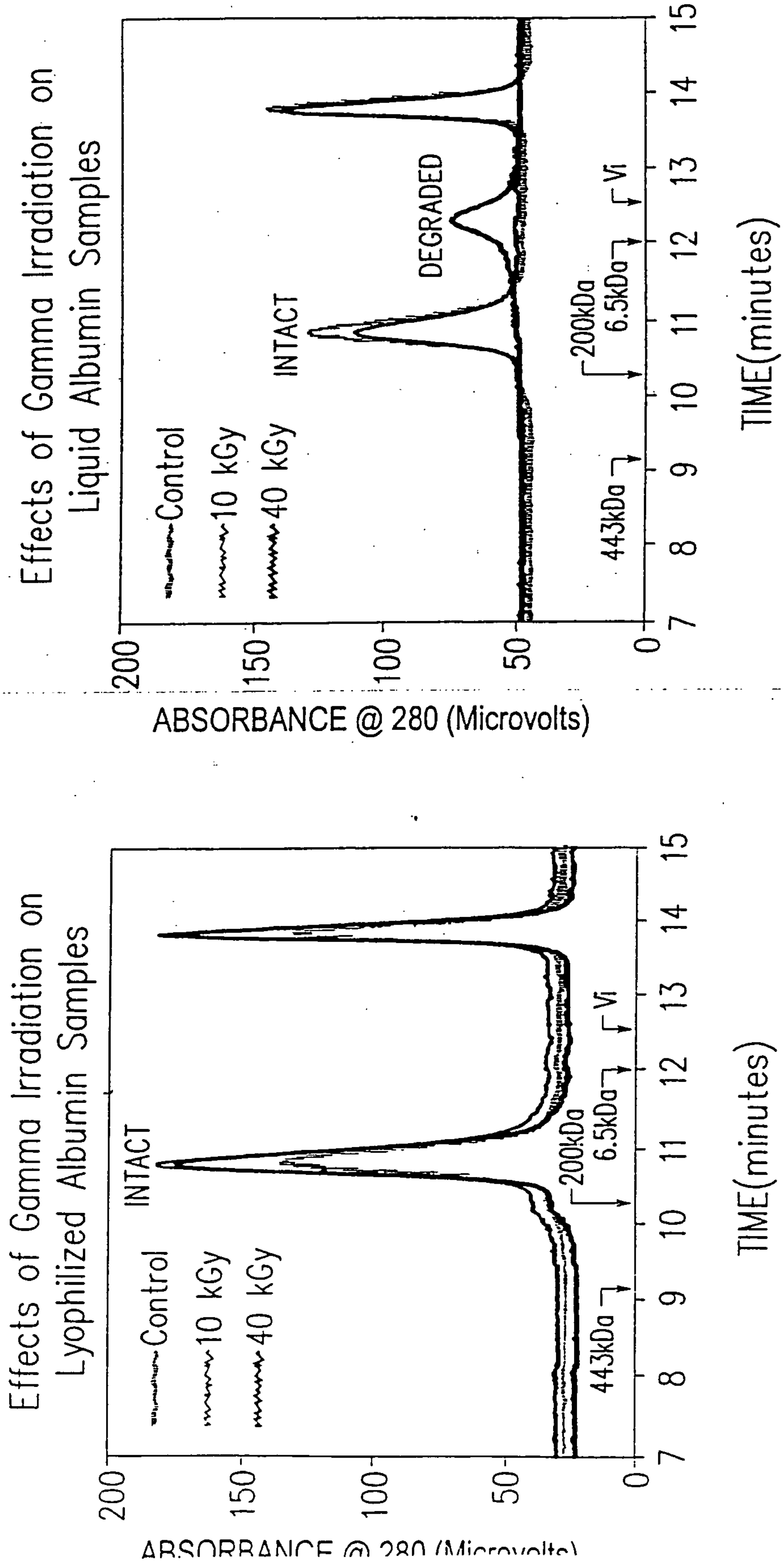

Effects of Gamma Irradiation on Lyophilized Albumin Samples
Control
10 kGy
40 kGy
INTACT
443kDa
200kDa
6.5kDa
Vi
ABSORBANCE @ 280 (Microvolts)
TIME(minutes)
Effects of Gamma Irradiation on Liquid Albumin Samples
Control
10 kGy
40 kGy
INTACT
DEGRADED
443kDa
200kDa
6.5kDa
Vi
ABSORBANCE @ 280 (Microvolts)
TIME(minutes)


Fig. 18

(121800rwa014)Effect of 45kGy Irradiation on Freeze Dried Thrombin (Primary Dry Cycle Only)
1%Albumin and +/- 200mM Ascorbate

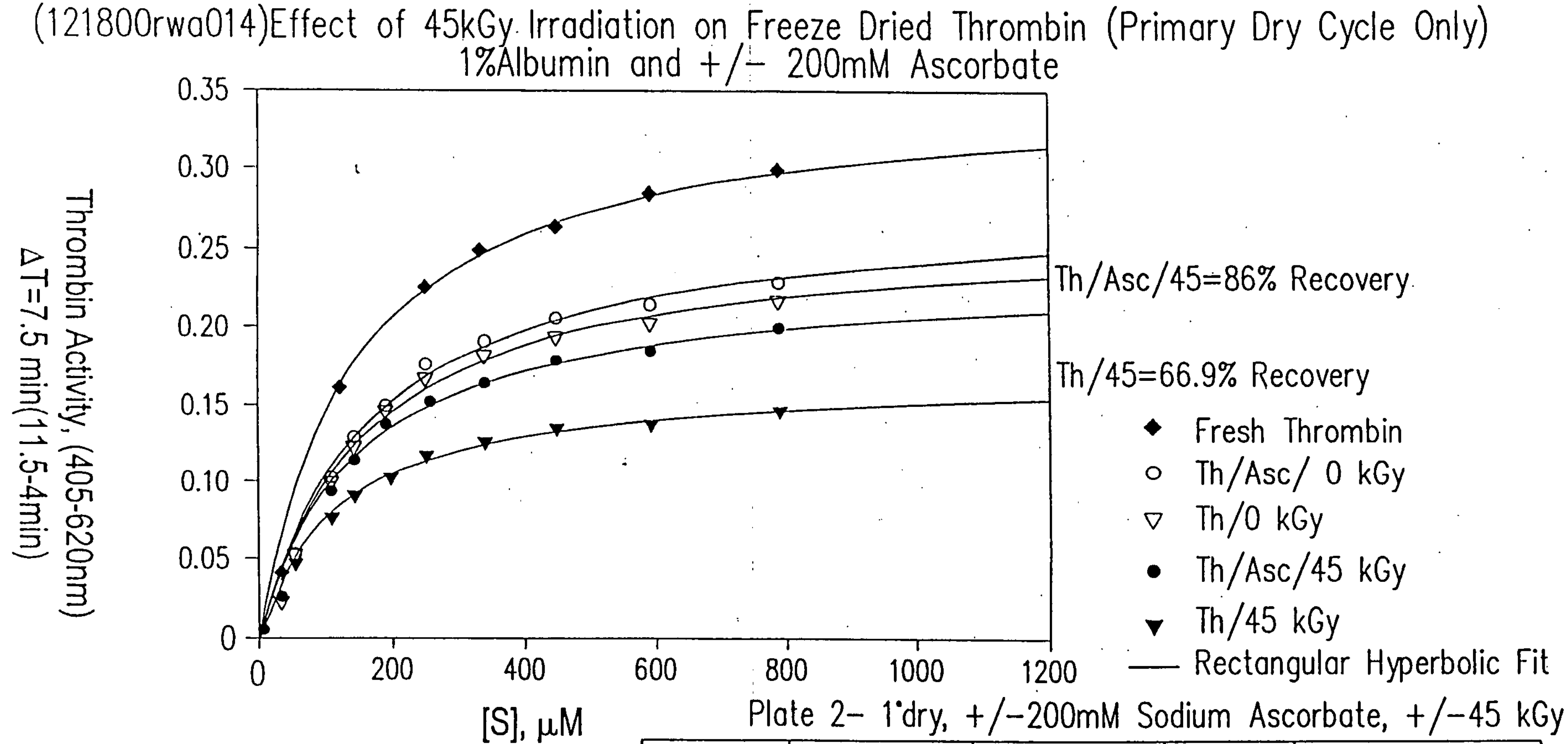


Plate 2- 1°dry, +/-200mM Sodium Ascorbate, +/-45 kGy

| Thrombin conditions | $K_m$, μM | $V_{max}$ Abs unit | $R^2$ | % moisture of Albumin moc vials |
|---|---|---|---|---|
| Th/1°/Asc/45 | 139.5226+/-8.7 | 0.2296+/-.0045 | 0.99175909 | 3.58+/-0.22 |
| Th/1°/Asc/0 | 161.2310+/-12.7 | 0.2740+/-0.0072 | 0.98839043 | 3.58+/-0.22 |
| Th/1°/45 | 111.3413+/-6.6 | 0.1641+/-0.0027 | 0.99155044 | 4.05+/-0.80 |
| Th/1°/0 | 153.3962+/-11.4 | 0.2566+/-0.0062 | 0.98925187 | 4.05+/-0.80 |
| Fresh Thrombin | 135.8572+/-6.06 | 0.3434+/-0.0041 | 0.99718607 | ------- |

Fig. 19

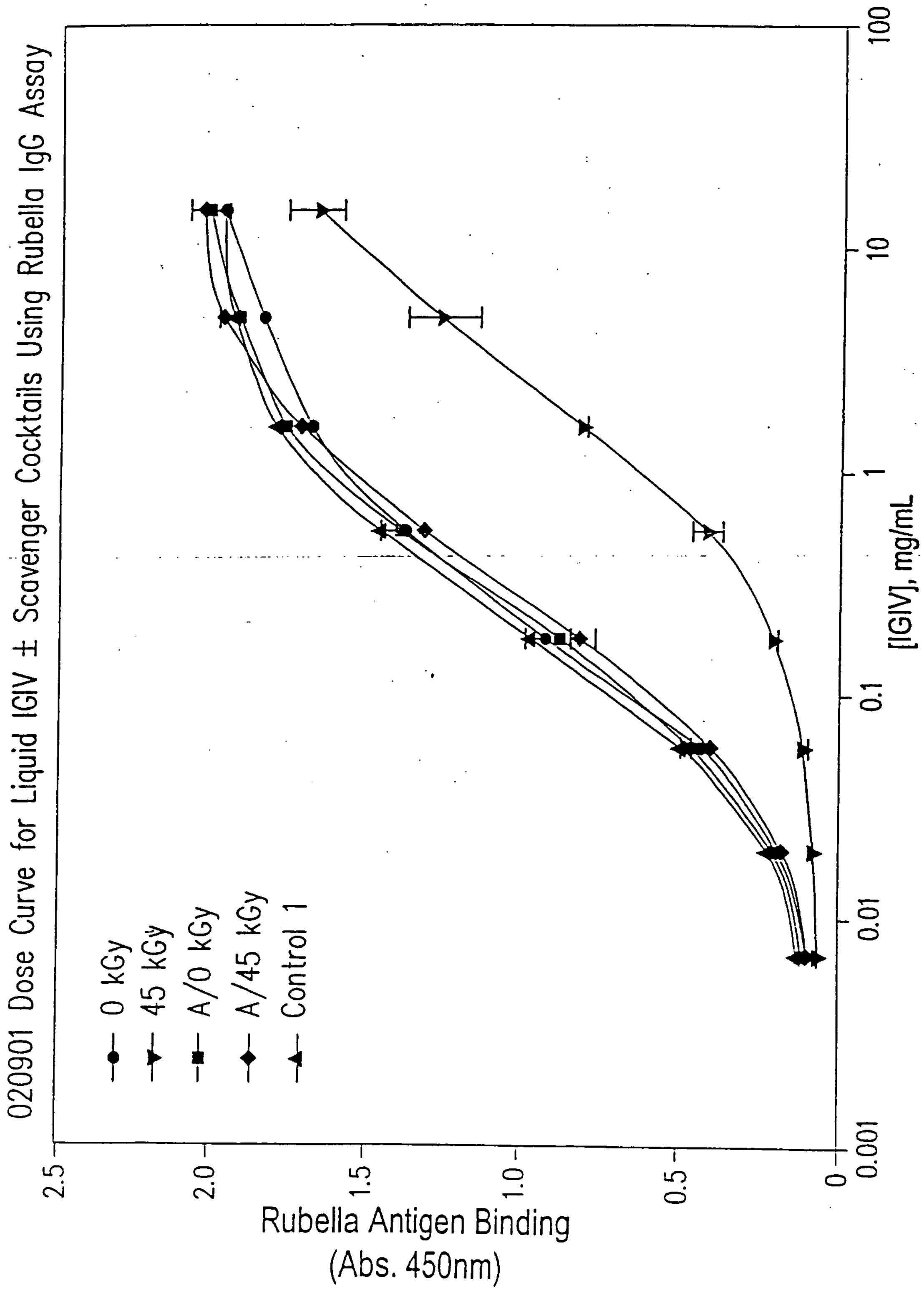
020901 Dose Curve for Liquid IGIV ± Scavenger Cocktails Using Rubella IgG Assay
0 kGy
45 kGy
A/0 kGy
A/45 kGy
Control 1
Rubella Antigen Binding
(Abs. 450nm)
2.5
2.0
1.5
1.0
0.5
0
0.001
0.01
0.1
1
10
100
[IGIV], mg/mL

Fig. 20

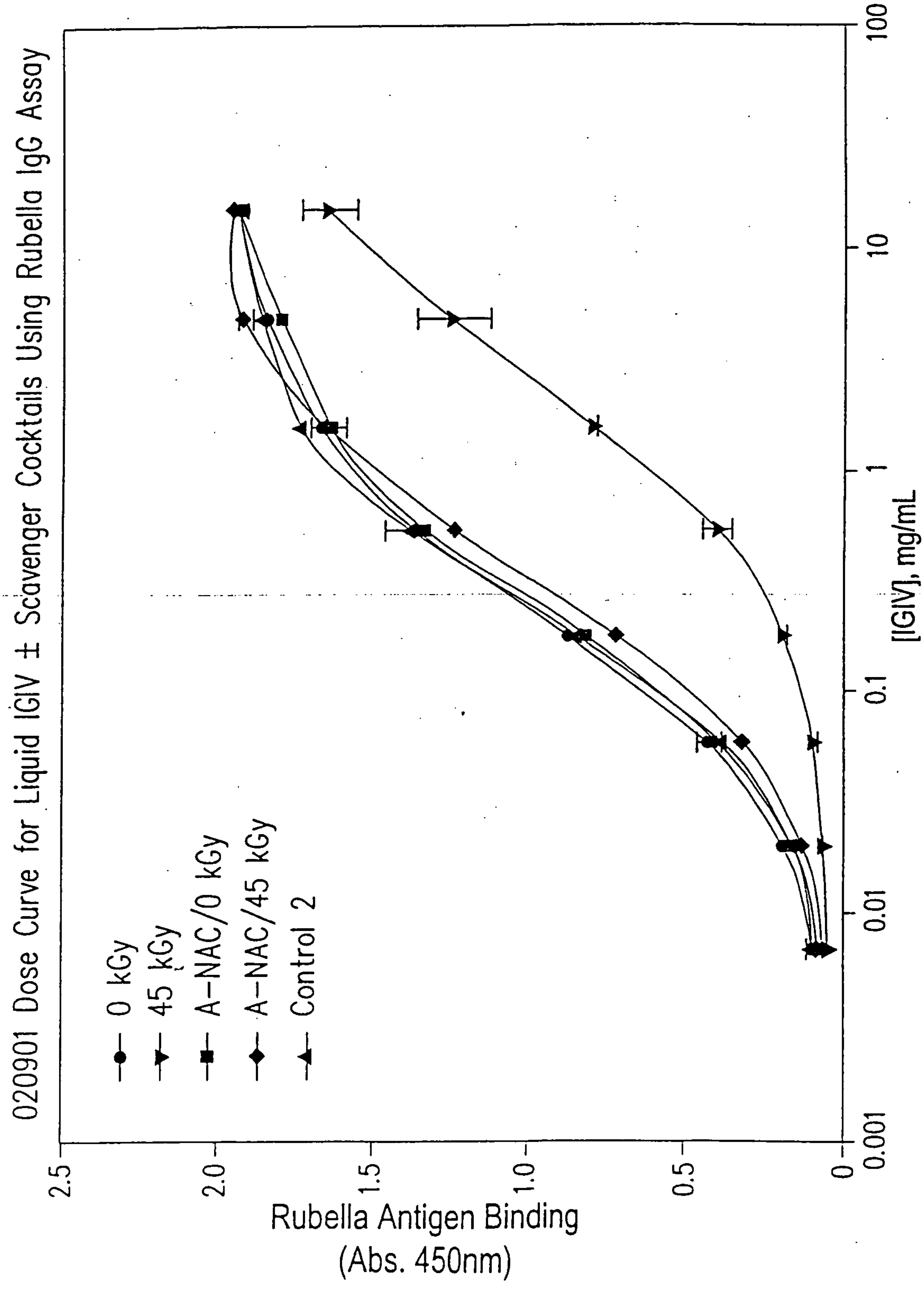
020901 Dose Curve for Liquid IGIV ± Scavenger Cocktails Using Rubella IgG Assay
0 kGy
45 kGy
A-NAC/0 kGy
A-NAC/45 kGy
Control 2
Rubella Antigen Binding
(Abs. 450nm)
2.5
2.0
1.5
1.0
0.5
0
0.001
0.01
0.1
1
10
100
[IGIV], mg/mL

Fig. 21

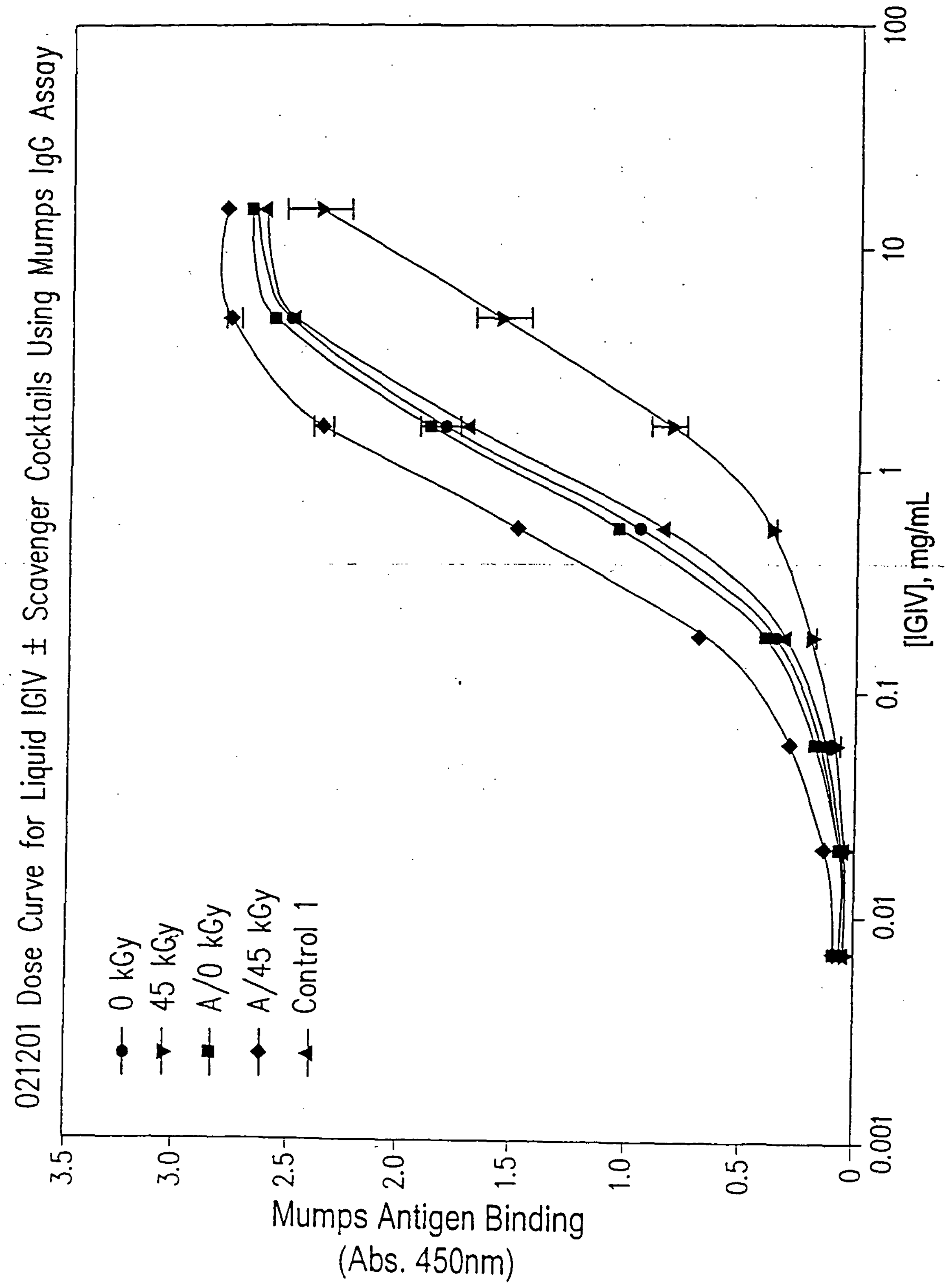
021201 Dose Curve for Liquid IGIV ± Scavenger Cocktails Using Mumps IgG Assay
0 kGy
45 kGy
A/0 kGy
A/45 kGy
Control 1
Mumps Antigen Binding
(Abs. 450nm)
3.5
3.0
2.5
2.0
1.5
1.0
0.5
0
0.001
0.01
0.1
1
10
100
[IGIV], mg/mL

Fig. 22

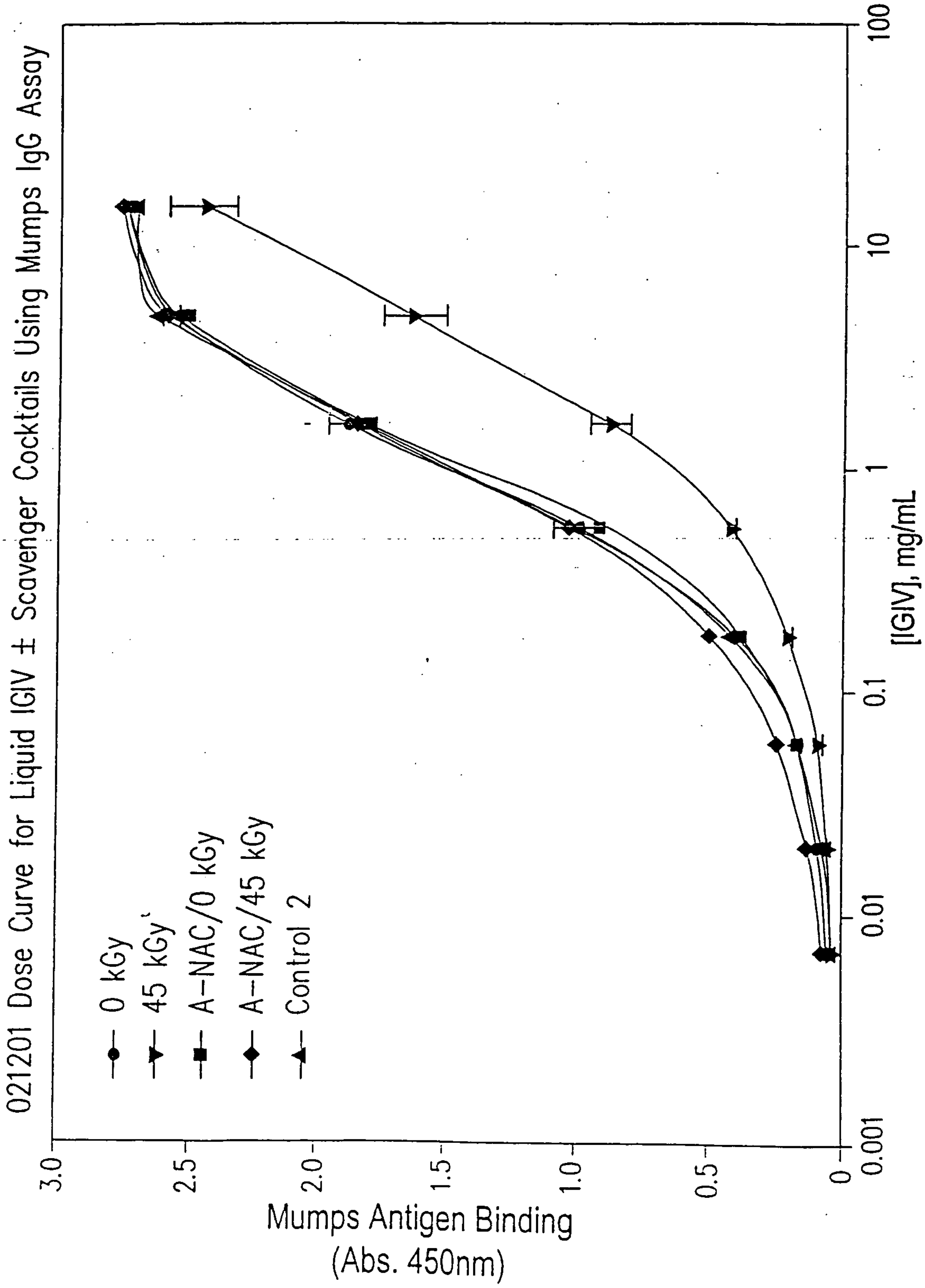
021201 Dose Curve for Liquid IGIV ± Scavenger Cocktails Using Mumps IgG Assay
0 kGy
45 kGy
A-NAC/0 kGy
A-NAC/45 kGy
Control 2
Mumps Antigen Binding (Abs. 450nm)
3.0
2.5
2.0
1.5
1.0
0.5
0
0.001
0.01
0.1
1
10
100
[IGIV], mg/mL

Fig. 23

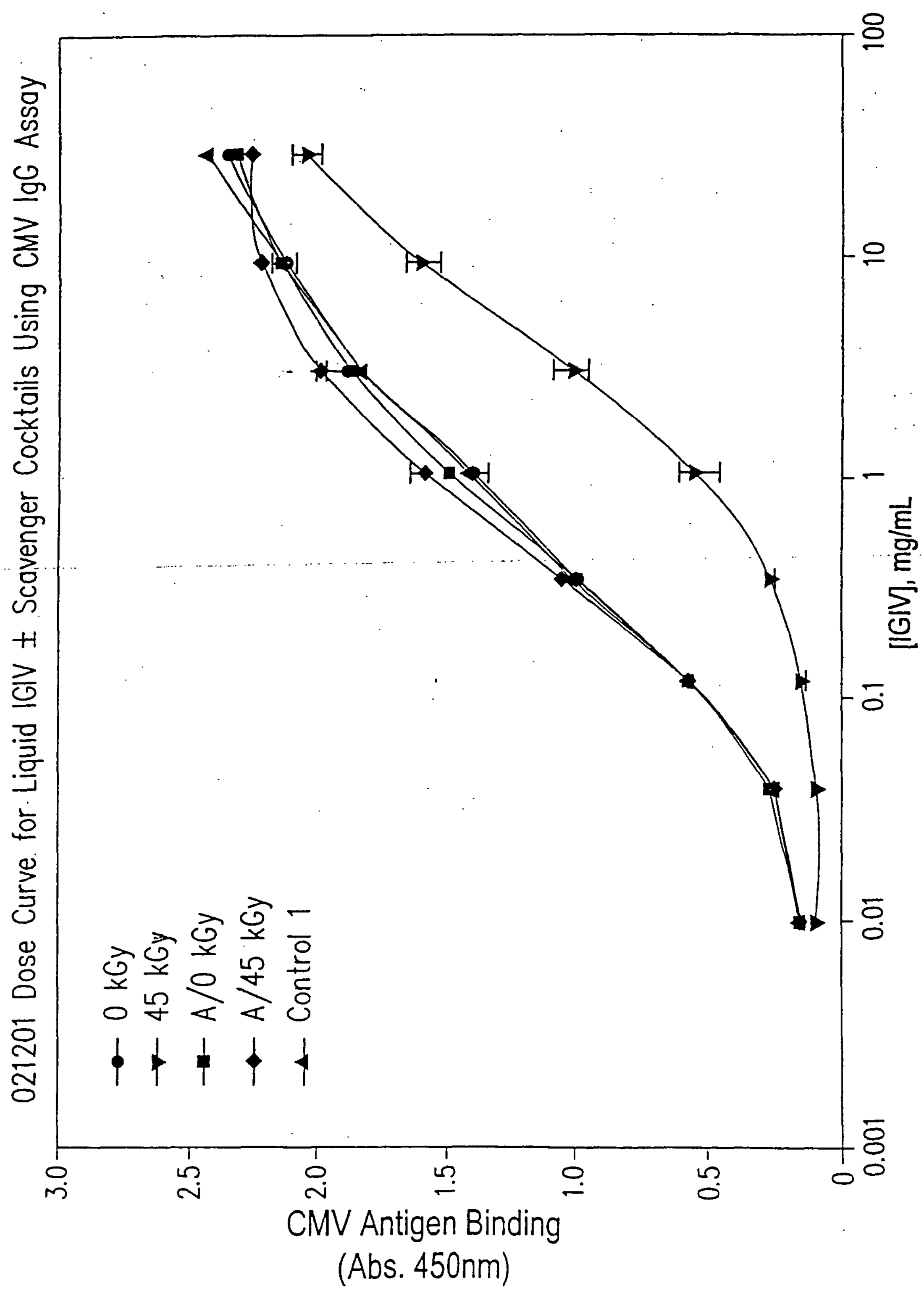
021201 Dose Curve for Liquid IGIV ± Scavenger Cocktails Using CMV IgG Assay
0 kGy
45 kGy
A/0 kGy
A/45 kGy
Control 1
CMV Antigen Binding
(Abs. 450nm)
3.0
2.5
2.0
1.5
1.0
0.5
0
0.001
0.01
0.1
1
10
100
[IGIV], mg/mL

Fig. 24

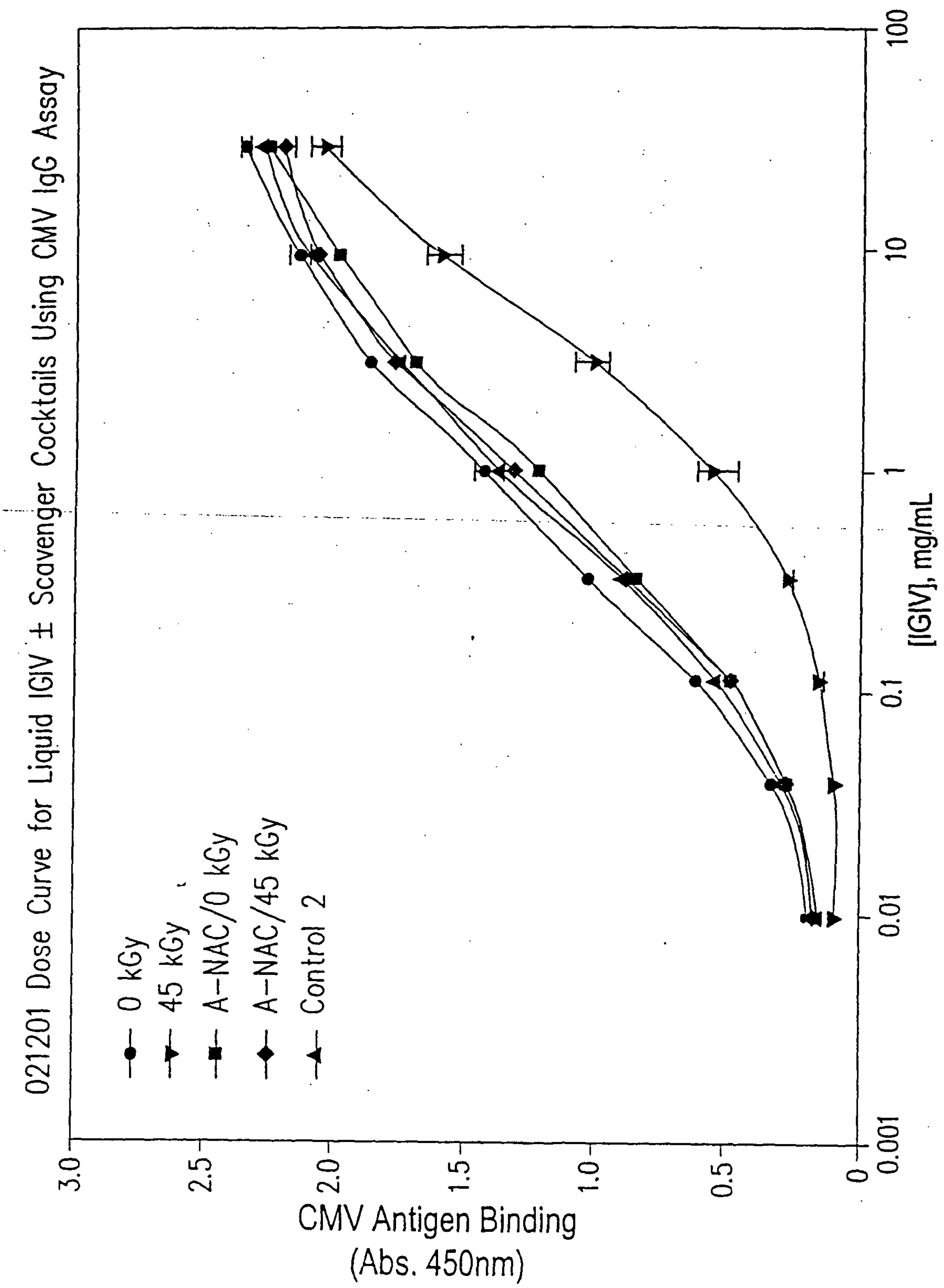
021201 Dose Curve for Liquid IGIV ± Scavenger Cocktails Using CMV IgG Assay
0 kGy
45 kGy
A-NAC/0 kGy
A-NAC/45 kGy
Control 2
CMV Antigen Binding
(Abs. 450nm)
3.0
2.5
2.0
1.5
1.0
0.5
0
0.001
0.01
0.1
1
10
100
[IGIV], mg/mL

Fig. 25

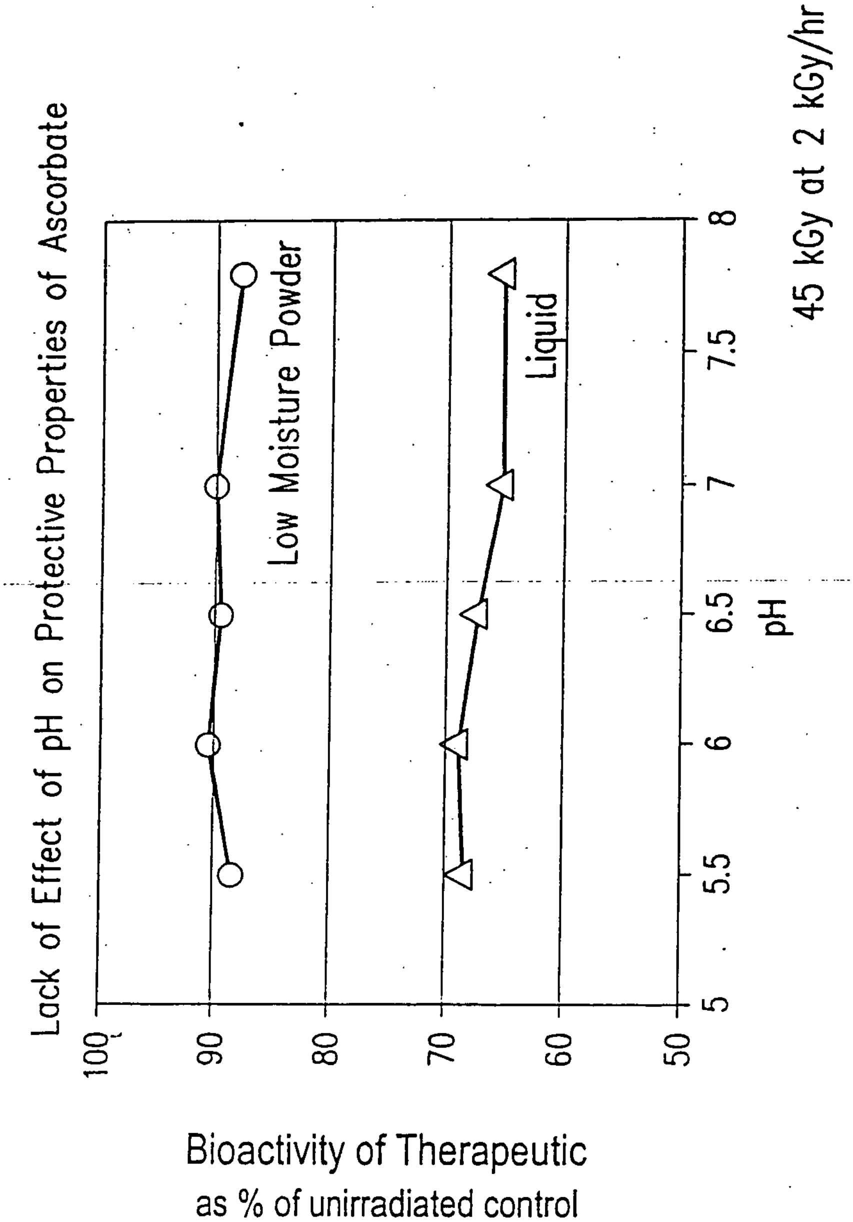
Lack of Effect of pH on Protective Properties of Ascorbate
Low Moisture Powder
Liquid
100
90
80
70
60
50
5
5.5
6
6.5
7
7.5
8
pH
45 kGy at 2 kGy/hr
Bioactivity of Therapeutic
as % of unirradiated control

Fig. 26

European Patent Office

# EUROPEAN SEARCH REPORT

Application Number
EP 06 02 0540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 730 933 A (DEPUY ORTHOPAEDICS, INC.) 24 March 1998 (1998-03-24) * column 5, line 53 - column 6, line 9; claims 1-18; examples 1-7 * | 1-26 | INV. A61L2/08 A61L2/00 |
| X | US 5 989 498 A (ST. JUDE MEDICAL, INC.) 23 November 1999 (1999-11-23) * column 6, line 47 - line 55; claims 1-20 * | 1 | |
| X | US 5 643 464 A (COLLAGEN CORP.) 1 July 1997 (1997-07-01) * column 6, line 19 - line 35; claims 1-4 * | 2 | |
| X | US 4 865 602 A (COLLAGEN CORP.) 12 September 1989 (1989-09-12) * the whole document * | 2 | |
| X | US 4 620 908 A (BIOCELL LABORATORIES, INC.) 4 November 1986 (1986-11-04) * the whole document * | 2 | TECHNICAL FIELDS SEARCHED (IPC) A61L |

The present search report has been drawn up for all claims

| Place of search | Date of completion of the search | Examiner |
|---|---|---|
| Munich | 14 November 2006 | Luethe, Herbert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

# ANNEX TO THE EUROPEAN SEARCH REPORT ON EUROPEAN PATENT APPLICATION NO. EP 06 02 0540

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2006

| Patent document cited in search report | | Publication date | Patent family member(s) | | | Publication date |
|---|---|---|---|---|---|---|
| US 5730933 | A | 24-03-1998 | NONE | | | |
| US 5989498 | A | 23-11-1999 | NONE | | | |
| US 5643464 | A | 01-07-1997 | US | 5527856 | A | 18-06-1996 |
| US 4865602 | A | 12-09-1989 | AU | 603744 | B2 | 22-11-1990 |
| | | | AU | 8068087 | A | 12-05-1988 |
| | | | CA | 1320451 | C2 | 20-07-1993 |
| | | | EP | 0270254 | A2 | 08-06-1988 |
| | | | ES | 2053562 | T3 | 01-08-1994 |
| | | | GR | 3007382 | T3 | 30-07-1993 |
| | | | JP | 1882498 | C | 10-11-1994 |
| | | | JP | 6002151 | B | 12-01-1994 |
| | | | JP | 63132664 | A | 04-06-1988 |
| | | | JP | 1901006 | C | 27-01-1995 |
| | | | JP | 5305133 | A | 19-11-1993 |
| | | | JP | 6022571 | B | 30-03-1994 |
| US 4620908 | A | 04-11-1986 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4620908 A


### Non-patent literature cited in the description


- **KEATHLY et al.** Is There Life After Irradiation? Part 2. *BioPharm,* July 1993
- **LEITMAN.** USe of Blood Cell Irradiation in the Prevention of Post Transfusion Graft-vs-Host Disease. *Transfusion Science,* 1989, vol. 10, 219-239
- **MEYER ; BOYD.** *Analytical Chem.,* 1959, vol. 31, 215-219
- **MAY et al.** *J. Biol. Standardization,* 1982, vol. 10, 249-259
- *Centers for Biologics Evaluation and Research,* 1990, 83-93
- The Merck Index. Merck & Co., Inc, 1989
- Martindale's Extra Pharmacopoeia. vol. 265, 1
- *Martindale's Extra Pharmacopecia,* vol. 265, 1
- **KAWAKISHI et al.** Radiation-Induced Degradation of D-glucose in Anaerobic Contition. *Agric. Biol. Chem.,* June 1977
- **GERGELY, J. et al.** Studies of Gama-Ray-Irradiated Human Immunoglobulin G. *SM-92/12*
- **KITCHEN et al.** Effect of Gamma Irradiation on the Human Immunodeficiency Virus and Human Coagulation Proteins. *Vox Sang,* 1989, vol. 56, 223-229
- **HIEMSTRA et al.** Inactivation of human immunodeficiency virus by gamma radiation and its effect on plasma and coagulation factors. *Transfusion,* 1991, vol. 31, 32-39
- **B.A. BRIDGES.** The effect of N-Ethylmaleimide on the radiation sensitivity of bacteria. *J. Gen. Microbiol.,* 1962, vol. 26, 467-472
- **JACOBS, G.P. ; SADEH, N.** Radiosensitization of Staphyloccocus aureus by p-hydroxybenzoic acid. *Int. J. Radiat. Biol.,* 1982, vol. 41, 351-356